# EUROPEAN PATENT APPLICATION

(11) **EP 4 289 957 A1**
(43) Date of publication of application: **13.12.2023**
(21) Application number: 22749829.2
(22) Date of filing: 04.02.2022
(51) Int. Cl.: C12N 15/52, C11C 3/00, C12N 1/15, C12N 1/19, C12N 1/21, C12N 5/10, C12N 9/00, C12N 15/12, C12P 7/64

(54) **ULTRA-LONG-CHAIN POLYUNSATURATED FATTY ACID COMPOSITION**

(30) Priority: 05.02.2021 JP 2021017675
(71) Applicant: Nissui Corporation, Tokyo 105-8676 (JP)
(72) Inventor: SEKIGUCHI, Takayoshi, Hachioji-shi, Tokyo 192-0991 (JP); TAKAHASHI, Misaki, Hachioji-shi, Tokyo 192-0991 (JP); EKO, Ryutaro, Hachioji-shi, Tokyo 192-0991 (JP); SATO, Seizo, Hachioji-shi, Tokyo 192-0991 (JP)
(74) Representative: Mewburn Ellis LLP
(86) International application number: PCT/JP2022/004520
(87) International publication number: WO 2022/168958

(57) **Abstract**

The purpose of the present invention is to provide very long chain polyunsaturated fatty acids and/or derivatives thereof. Substance production of very long chain polyunsaturated fatty acids and/or derivatives thereof has been possible by the conventional technology. However, concentrations of the produced substances have been low. The substance production of very long chain polyunsaturated fatty acids and/or derivatives thereof has been possible. However, the substances produced cannot be obtained in sufficient concentrations and cannot be used as compositions. The present invention provides very long chain polyunsaturated fatty acids and/or derivatives thereof with increased concentrations. Moreover, the object of the present invention is to provide a composition comprising very long chain polyunsaturated fatty acids and/or derivatives thereof.

## Description

### Technical Field

The present invention relates to the technical field of microbial oils comprising very long chain polyunsaturated fatty acids and/or derivatives thereof, and a method for their production.

### Background Art

Very long chain polyunsaturated fatty acids (VLC-PUFAs) are polyunsaturated fatty acids having more carbons than long chain polyunsaturated fatty acids such as DHA and EPA. A definition of very long chain unsaturated fatty acids is not clearly defined, but they may be defined as long chain unsaturated fatty acids of at least C26, at least C28, or at least C30. Although very long chain unsaturated fatty acids account for only a small proportion of lipids that make up living organisms, they play important physiological and pathological roles. Studies on Stargardt's disease, an inherited macular degeneration, and analyses of knockout mice have revealed that very long chain unsaturated fatty acids are involved in retinal photoreceptor function and skin barrier formation. In addition, C28-C38 very long chain unsaturated fatty acids are reported to be synthesized by Elongation of very long chain fatty acids 4 (ELOVL4) (Non-patent Document 1: PNAS (2008) vol. 105, No. 35, 12843-12848). In mammals, ELOVL4 is highly expressed in retina and is also expressed in brain, skin, and testis. Expression analysis of ELOVL4 involved in elongation of VLC-PUFAs in zebrafish embryogenesis has been reported (Non-patent Document 2: Biochimica et Biophysica Acta 1801 (2010) 1145-1154). An elovl4b gene of black seabream has been cloned and expressed in yeast to functionally characterize ELOVL4b (Non-patent Document 3: Comparative Biochemistry and Physiology, Part B 212 (2017) 41-50). An elovl4a gene of African sharptooth catfish has been cloned and expressed in yeast to functionally characterize ELOVL4a (Non-patent Document 4: Lipids (2017) 52: 837-848).

It has been shown that ω-3 VLC-PUFAs, which are a type of very long chain unsaturated fatty acids VLC-PUFAs, have anti-inflammatory effects, protective effects on cells and tissues, and inhibitory effects on age-related cellular senescence (Patent Document 1: US 2020/0009100 A).

The following methods for producing very long chain unsaturated fatty acids have been reported: a method of extracting from fish oil (Patent Document 2: WO2020/117070), a method of extracting from natural oils such as fish oil, squid oil, algae oil, and krill oil (Patent Document 3: JP 2018-514644 T), and a method for producing VLC-PUFAs by chemical synthesis (Patent Document 4: WO2011/053892). A method of transducing elongases and desaturases are transduced into cells has been reported (Patent Document 5: JP 2012-509059 T, Non-patent Document 5: PNAS December 4, 2001 vol. 98 no. 25; 14304-14309). In addition, a method of transducing ELOVL4 genes into mammalian cells (Patent Document 3: US 2012/0071558), a method of transducing rat and mouse ELOVL4 into cultured cells (Non-patent Document 1: PNAS (2008) vol. 105, No. 35, 12843-12848), and a method of transducing ELOVL4 into yeast (Non-patent Document 6: Polyunsaturated Fatty Acid Metabolism. 2018, Pages 31-60) have been reported.

### Citation List

### Patent Literature

Patent Document 1: US 2020/0009100 A
Patent Document 2: WO 2020/117070
Patent Document 3: JP 2018-514644 A
Patent Document 4: WO 2011/053892
Patent Document 5: JP 2012-509059 A

### Non-patent Document

Non-patent Document 1: PNAS (2008) vol. 105, No. 35, 12843-12848
Non-patent Document 2: Biochimica et Biophysica Acta 1801 (2010) 1145-1154
Non-patent Document 3: Comparative Biochemistry and Physiology, Part B 212 (2017) 41-50
Non-patent Document 4: Lipids (2017) 52: 837-848
Non-patent Document 5: PNAS December 4, 2001 vol. 98 no. 25; 14304-14309
Non-patent Document 6: Polyunsaturated Fatty Acid Metabolism. 2018, Pages 31-60

### Summary of Invention

### Technical Problem

This disclosure provides very long chain polyunsaturated fatty acids and/or derivatives thereof. Substance production of very long chain polyunsaturated fatty acids and/or derivatives thereof has been possible by the conventional technology. However, concentrations of the produced substances have been low. To be specific, attempts to produce microbial oils comprising VLC-PUFAs according to the prior art have shown that a concentration of total VLC-PUFAs in microbial oils is less than 0.8% and a concentration of each VLC-PUFA is less than 0.5%. As described above, the conventional technology has made it possible to produce substances for very long chain polyunsaturated fatty acids and/or derivatives. However, the substances produced have not been obtained in sufficient concentrations to be used as compositions.

Thus, this disclosure provides high concentrations of very long chain polyunsaturated fatty acids and/or derivatives thereof, and further provides compositions comprising very long chain polyunsaturated fatty acids and/or derivatives thereof.

### Solution to Problem

The inventors have conducted intensive research on cells that produce very long chain unsaturated fatty acids and culture conditions of the cells, and have found that very long chain polyunsaturated fatty acids can be produced by culturing cells transduced with polyunsaturated fatty acid (PUFA) chain elongase genes and inducing expression of the PUFA chain elongase genes, resulting in the present invention. Thus, the present invention relates to the following.
[1] A composition comprising a cell expressing a PUFA chain elongase and/or a crushed material thereof, and a polyunsaturated fatty acid having at least 30 carbons and/or a derivative thereof.
[2] The composition according to item 1, wherein the polyunsaturated fatty acid having at least 30 carbons comprises at least one polyunsaturated fatty acid selected from the group consisting of the following:
   (1) a polyunsaturated fatty acid having 30 carbons;
   (2) a polyunsaturated fatty acid having 32 carbons;
   (3) a polyunsaturated fatty acid having 34 carbons; and
   (4) a polyunsaturated fatty acid having 36 carbons.
[3] The composition according to item 2, wherein a content of one polyunsaturated fatty acid selected from the group consisting of (1) to (4) and/or a derivative thereof is at least 0.5 area%.
[4] The composition according to item 2 or 3, wherein a content of the polyunsaturated fatty acids of (1) to (4) and/or derivatives thereof is at least 1.0 area%, at least 1.5 area%, at least 2.0 area%, or at least 2.5 area%.
[5] The composition according to any one of items 2 to 4, wherein a ratio of a content of stearic acid and/or a derivative thereof to a total content of the polyunsaturated fatty acids of (1) to (4) and/or the derivatives thereof is less than 6.0, less than 5.0, less than 2.5, or less than 2.0.
[6] The composition according to any one of items 2 to 5, wherein the polyunsaturated fatty acid and/or the derivative thereof in the composition satisfies at least one of the following conditions:
   (1) a ratio of a content of stearic acid and/or a derivative thereof to a content of the polyunsaturated fatty acid having 30 carbons and/or the derivative thereof is less than 50, less than 30, less than 20, or less than 10;
   (2) a ratio of a content of stearic acid and/or a derivative thereof to a content of the polyunsaturated fatty acid having 32 carbons and/or the derivative thereof is less than 15, less than 10, or less than 5;
   (3) a ratio of a content of stearic acid and/or a derivative thereof to a content of the polyunsaturated fatty acid having 34 carbons and/or the derivative thereof is less than 10, less than 5, or less than 2; and
   (4) a ratio of a content of stearic acid and/or a derivative thereof to a content of the polyunsaturated fatty acid having 36 carbons and/or the derivative thereof is less than 20.
[7] The composition according to any one of items 1 to 6, comprising an impurity having a chemical formula of C23H36O3.
[8] The composition according to any one of items 1 to 7, wherein a content of a cholesterol ester, phospholipid, glycolipid, monoglyceride, diglyceride, and triglyceride is at least 70 wt.% of a total weight of the composition.
[9] The composition according to any one of items 2 to 8, wherein at least one fatty acid of a constituent fatty acid of a cholesterol ester, phospholipid, glycolipid, monoglyceride, diglyceride, and triglyceride is at least one polyunsaturated fatty acid of (1) to (4).
[10] The composition according to any one of items 2 to 9, wherein at least one fatty acid of constituent fatty acids of a cholesterol ester, phospholipid, glycolipid, monoglyceride, diglyceride, and triglyceride is at least one polyunsaturated fatty acid of (1) to (4), and of the constituent fatty acids of a cholesterol ester, phospholipid, glycolipid, monoglyceride, diglyceride, and triglyceride, a fatty acid other than the polyunsaturated fatty acids of (1) to (4) is a fatty acid having from 8 to 22 hydrocarbons.
[11] The composition according to any one of items 1 to 10, comprising a PUFA having at least 0.5 area%.
[12] The composition according to item 11, wherein the PUFA comprises a non-productive PUFA externally added.
[13] The composition according to any one of items 1 to 12, wherein the cell is yeast.
[14] The composition according to any one of items 1 to 13, wherein the PUFA chain elongase is one of the following:
   a) non-mammalian ELOVL4; and
   b) a protein comprising an amino acid sequence having at least 90% sequence identity to an amino acid sequence encoding non-mammalian ELOVL4 and having PUFA chain elongation activity.
[15] The composition according to any one of items 1 to 14, wherein a gene of the PUFA chain elongase is expressed by an inducible promoter.
[16] Crude oil comprising a polyunsaturated fatty acid having at least 30 carbons and/or a derivative thereof at a content of at least 1.0 area%, at least 1.5 area%, at least 2.0 area%, or at least 2.5 area%.
[17] The crude oil according to item 16, wherein the polyunsaturated fatty acid having at least 30 carbons comprises at least one polyunsaturated fatty acid selected from the group consisting of the following:
   (1) a polyunsaturated fatty acid having 30 carbons;
   (2) a polyunsaturated fatty acid having 32 carbons;
   (3) a polyunsaturated fatty acid having 34 carbons; and
   (4) a polyunsaturated fatty acid having 36 carbons.
[18] The crude oil according to item 17, wherein a content of one polyunsaturated fatty acid selected from the group consisting of (1) to (4) and/or a derivative thereof is at least 0.5 area%.
[19] The crude oil according to item 17 or 18, wherein a content of the polyunsaturated fatty acids of (1) to (4) and/or derivatives thereof is at least 1.0 area%, at least 1.5 area%, at least 2.0 area%, or at least 2.5 area%.
[20] The crude oil according to any one of items 17 to 19, wherein a ratio of a content of stearic acid and/or a derivative thereof to a total content of the polyunsaturated fatty acids of (1) to (4) and/or the derivatives thereof is less than 6.0, less than 5.0, less than 2.5, or less than 2.0.
[21] The crude oil according to any one of items 16 to 20, wherein the polyunsaturated fatty acid and/or the derivative thereof in the crude oil satisfies at least one of the following conditions:
   (1) a ratio of a content of stearic acid and/or a derivative thereof to a content of the polyunsaturated fatty acid having 30 carbons and/or the derivative thereof is less than 50, less than 30, less than 20, or less than 10;
   (2) a ratio of a content of stearic acid and/or a derivative thereof to a content of the polyunsaturated fatty acid having 32 carbons and/or the derivative thereof is less than 15, less than 10, or less than 5;
   (3) a ratio of a content of stearic acid and/or a derivative thereof to a content of the polyunsaturated fatty acid having 34 carbons and/or the derivative thereof is less than 10, less than 5, or less than 2; and
   (4) a ratio of a content of stearic acid and/or a derivative thereof to a content of the polyunsaturated fatty acid having 36 carbons and/or the derivative thereof is less than 20.
[22] The crude oil according to any one of items 16 to 21, comprising an impurity having a chemical formula of C23H36O3.
[23] The crude oil according to any one of items 16 to 22, wherein a content of a cholesterol ester, phospholipid, glycolipid, monoglyceride, diglyceride, and triglyceride is at least 70 wt.% of a total weight.
[24] The crude oil according to any one of items 17 to 23, wherein at least one fatty acid of a constituent fatty acid of a cholesterol ester, phospholipid, glycolipid, monoglyceride, diglyceride, and triglyceride is at least one polyunsaturated fatty acid of (1) to (4).
[25] The crude oil according to any one of items 17 to 24, wherein at least one fatty acid of constituent fatty acids of a cholesterol ester, phospholipid, glycolipid, monoglyceride, diglyceride, and triglyceride is at least one polyunsaturated fatty acid of (1) to (4), and of the constituent fatty acids of a cholesterol ester, phospholipid, glycolipid, monoglyceride, diglyceride, and triglyceride, a fatty acid other than the polyunsaturated fatty acids of (1) to (4) is a fatty acid having from 8 to 22 hydrocarbons.
[26] The crude oil according to any one of items 16 to 25, comprising a PUFA having at least 0.5 area%.
[27] The crude oil according to item 26, wherein the PUFA comprises a non-productive PUFA externally added.
[28] The crude oil according to any one of items 16 to 27, wherein the crude oil is a crude oil derived from yeast.
[29] The crude oil according to item 28, wherein the yeast is a yeast expressing a PUFA chain elongase gene.
[30] The crude oil according to item 29, wherein the PUFA chain elongase gene is expressed by an inducible promoter.
[31] The crude oil according to item 29 or 30, wherein the PUFA chain elongase gene comprises:
   a) a polynucleotide encoding non-mammalian ELOVL4; or
   b) a polynucleotide having a nucleotide sequence having at least 90% sequence identity to a nucleotide sequence of the polynucleotide encoding non-mammalian ELOVL4 and encoding a protein having PUFA chain elongation activity.
[32] A composition comprising a polyunsaturated fatty acid having at least 30 carbons and/or a derivative thereof, wherein a content of the polyunsaturated fatty acid and/or the derivative thereof is at least 1.0 area%, at least 1.5 area%, at least 2.0 area%, or at least 2.5 area%, and a total content of an acylcholesterol, phospholipid, glycolipid, monoglyceride, diglyceride, and triglyceride is at least 70 wt.%.
[33] The composition according to item 32, wherein the polyunsaturated fatty acid having at least 30 carbons comprises at least one polyunsaturated fatty acid selected from the group consisting of the following:
   (1) a polyunsaturated fatty acid having 30 carbons;
   (2) a polyunsaturated fatty acid having 32 carbons;
   (3) a polyunsaturated fatty acid having 34 carbons; and
   (4) a polyunsaturated fatty acid having 36 carbons.
[34] The composition according to item 33, wherein a content of one polyunsaturated fatty acid selected from the group consisting of (1) to (4) and/or a derivative thereof is at least 0.5 area%.
[35] The composition according to item 33 or 34, comprising the polyunsaturated fatty acid having at least 30 carbons and/or the derivative thereof, wherein the polyunsaturated fatty acid and/or the derivative thereof comprises the polyunsaturated fatty acids of (1) to (4) and/or derivatives thereof at a content of at least 1.0 area%, at least 1.5 area%, at least 2.0 area%, or at least 2.5 area%.
[36] The composition according to any one of items 33 to 35, wherein a ratio of a content of stearic acid and/or a derivative thereof to a total content of the polyunsaturated fatty acids of (1) to (4) and/or the derivatives thereof is less than 6.0, less than 5.0, less than 2.5, or less than 2.0.
[37] The composition according to any one of items 33 to 36, wherein the polyunsaturated fatty acid and/or the derivative thereof in the composition satisfies at least one of the following conditions:
   (1) a ratio of a content of stearic acid and/or a derivative thereof to a content of the polyunsaturated fatty acid having 30 carbons and/or the derivative thereof is less than 50, less than 30, less than 20, or less than 10;
   (2) a ratio of a content of stearic acid and/or a derivative thereof to a content of the polyunsaturated fatty acid having 32 carbons and/or the derivative thereof is less than 15, less than 10, or less than 5;
   (3) a ratio of a content of stearic acid and/or a derivative thereof to a content of the polyunsaturated fatty acid having 34 carbons and/or the derivative thereof is less than 10, less than 5, or less than 2; and
   (4) a ratio of a content of stearic acid and/or a derivative thereof to a content of the polyunsaturated fatty acid having 36 carbons and/or the derivative thereof is less than 20.
[38] The composition according to any one of items 33 to 37, comprising an impurity of a chemical formula of C23H36O3.
[39] The composition according to any one of items 32 to 38, wherein a content of a cholesterol ester, phospholipid, glycolipid, monoglyceride, diglyceride, and triglyceride is at least 70 wt.% of a total weight of the composition.
[40] The composition according to any one of items 33 to 39, wherein at least one fatty acid of a constituent fatty acid of a cholesterol ester, phospholipid, glycolipid, monoglyceride, diglyceride, and triglyceride is at least one polyunsaturated fatty acid of (1) to (4).
[41] The composition according to any one of items 33 to 40, wherein at least one fatty acid of constituent fatty acids of a cholesterol ester, phospholipid, glycolipid, monoglyceride, diglyceride and triglyceride is at least one polyunsaturated fatty acid of (1) to (4), and of the constituent fatty acids of a cholesterol ester, phospholipid, glycolipid, monoglyceride, diglyceride, and triglyceride, a fatty acid other than the polyunsaturated fatty acids of (1) to (4) is a fatty acid having from 8 to 22 hydrocarbons.
[42] The composition according to any one of items 32 to 41, comprising a PUFA having at least 0.5 area%.
[43] The composition according to item 42, wherein the PUFA comprises a non-productive PUFA externally added.
[44] Microbial oil comprising a polyunsaturated fatty acid having at least 30 carbons and/or a derivative thereof at a content of at least 1.0 area%, at least 1.5 area%, at least 2.0 area%, or at least 2.5 area%.
[45] The microbial oil according to item 44 wherein the polyunsaturated fatty acid having at least 30 carbons comprises at least one polyunsaturated fatty acid selected from the group consisting of the following:
   (1) a polyunsaturated fatty acid having 30 carbons;
   (2) a polyunsaturated fatty acid having 32 carbons;
   (3) a polyunsaturated fatty acid having 34 carbons; and
   (4) a polyunsaturated fatty acid having 36 carbons.
[46] The microbial oil according to item 45, wherein a content of one polyunsaturated fatty acid selected from the group consisting of (1) to (4) and/or a derivative thereof is at least 0.5 area%.
[47] The microbial oil according to item 45 or 46, comprising the polyunsaturated fatty acid having at least 30 carbons and/or the derivative thereof, wherein the polyunsaturated fatty acid and/or the derivative thereof comprises the polyunsaturated fatty acids of (1) to (4) and/or derivatives thereof at a content of at least 1.0 area%, at least 1.5 area%, at least 2.0 area%, or at least 2.5 area%.
[48] The microbial oil according to any one of items 45 to 47, wherein a ratio of a content of stearic acid and/or a derivative thereof to a total content of the polyunsaturated fatty acids of (1) to (4) and/or the derivatives thereof in the microbial oil is less than 6.0, less than 5.0, less than 2.5, or less than 2.0.
[49] The microbial oil according to any one of items 45 to 48, wherein the polyunsaturated fatty acid and/or the derivative thereof in the microbial oil satisfies at least one of the following conditions:
   (1) a ratio of a content of stearic acid and/or a derivative thereof to a content of the polyunsaturated fatty acid having 30 carbons and/or the derivative thereof is less than 50, less than 30, less than 20, or less than 10;
   (2) a ratio of a content of stearic acid and/or a derivative thereof to a content of the polyunsaturated fatty acid having 32 carbons and/or the derivative thereof is less than 15, less than 10, or less than 5;
   (3) a ratio of a content of stearic acid and/or a derivative thereof to a content of the polyunsaturated fatty acid having 34 carbons and/or the derivative thereof is less than 10, less than 5, or less than 2; and
   (4) a ratio of a content of stearic acid and/or a derivative thereof to a content of the polyunsaturated fatty acid having 36 carbons and/or the derivative thereof is less than 20.
[50] The microbial oil according to any one of items 44 to 48, comprising an impurity having a chemical formula of C23H36O3.
[51] The microbial oil according to any one of items 44 to 50, wherein a content of a cholesterol ester, phospholipid, glycolipid, monoglyceride, diglyceride, and triglyceride is at least 70 wt.% of a total weight.
[52] The microbial oil according to any one of items 44 to 51, wherein a content of a cholesterol ester, phospholipid, glycolipid, monoglyceride, diglyceride, and triglyceride is at least 70 wt.% of a total weight.
[53] The microbial oil according to any one of items 45 to 52, wherein at least one fatty acid of a constituent fatty acid of a cholesterol ester, phospholipid, glycolipid, monoglyceride, diglyceride, and triglyceride is at least one polyunsaturated fatty acid of (1) to (4).
[54] The microbial oil according to any one of items 45 to 53, wherein at least one fatty acid of constituent fatty acids of a cholesterol ester, phospholipid, glycolipid, monoglyceride, diglyceride, and triglyceride is at least one polyunsaturated fatty acid of (1) to (4), and of the constituent fatty acids of a cholesterol ester, phospholipid, glycolipid, monoglyceride, diglyceride, and triglyceride, a fatty acid other than the polyunsaturated fatty acids of (1) to (4) is a fatty acid having from 8 to 22 hydrocarbons.
[55] The microbial oil according to any one of items 44 to 54, comprising a PUFA having at least 0.5 area%.
[56] The microbial oil according to item 55, wherein the PUFA comprises a non-productive PUFA externally added.
[57] The microbial oil according to any one of items 44 to 56, wherein the microbial oil is crude oil.
[58] The microbial oil according to any one of items 44 to 57, wherein the microbial oil is yeast oil.
[59] The microbial oil according to item 58, wherein the yeast oil is an oil derived from yeast expressing a PUFA chain elongase gene.
[60] The microbial oil according to item 59, wherein the PUFA chain elongase gene is expressed by an inducible promoter.
[61] The microbial oil according to item 59 or 60, wherein the PUFA chain elongase gene comprises:
   a) a polynucleotide encoding non-mammalian ELOVL4; or
   b) a polynucleotide having a nucleotide sequence having at least 90% sequence identity to a nucleotide sequence of the polynucleotide encoding non-mammalian ELOVL4 and encoding a protein having PUFA chain elongation activity.
[62] A method for producing a polyunsaturated fatty acid and/or a derivative thereof, the method comprising
   (A) transducing an expression system of a PUFA chain elongase into a cell,
   (B) conditioning the cell transduced to a state of high VLC-PUFA content, and
   (C) inducing expression of the PUFA chain elongase in the cell conditioned.
[63] The method according to item 62, wherein the cell is yeast.
[64] The method according to item 62 or 63, wherein the PUFA chain elongase is non-mammalian ELOVL4.
[65] The method according to any one of items 62 to 64, wherein a gene of the PUFA chain elongase is expressed by an inducible promoter.
[66] The method according to item 65, wherein the inducible promoter is galactose-inducible and an addition of galactose induces an expression of an elovl4 gene.
[67] The method according to any one of items 62 to 66, wherein step (B) is culturing in a culture medium comprising a polyunsaturated fatty acid having a carbon chain length of 18 to 22 and/or a derivative thereof.
[68] The method according to any one of items 62 to 67, wherein the expression of the PUFA chain elongase is induced in the culture solution having a turbidity value in a range of 1.5 to 11.
[69] The method according to any one of items 62 to 68, wherein a polyunsaturated fatty acid to be produced comprises a polyunsaturated fatty acid having at least 30 carbons and/or a derivative thereof.
[70] The method according to item 69, wherein the polyunsaturated fatty acid having at least 30 carbons comprises at least one polyunsaturated fatty acid selected from the group consisting of the following:
   (1) a polyunsaturated fatty acid having 30 carbons;
   (2) a polyunsaturated fatty acid having 32 carbons;
   (3) a polyunsaturated fatty acid having 34 carbons; and
   (4) a polyunsaturated fatty acid having 36 carbons.
[71] The method according to item 70, wherein a content of one polyunsaturated fatty acid selected from the group consisting of (1) to (4) and/or a derivative thereof is at least 0.5 area%.
[72] The method according to item 70 or 71, wherein the polyunsaturated fatty acid having at least 30 carbons and/or the derivative thereof comprises the polyunsaturated fatty acids of (1) to (4) and/or the derivatives thereof at a content of at least 1.0 area%, at least 1.5 area%, at least 2.0 area%, or at least 2.5 area%.
[73] The method according to any one of items 70 to 72, wherein a ratio of a content of stearic acid and/or a derivative thereof to a total content of the polyunsaturated fatty acids of (1) to (4) and/or the derivatives thereof is less than 6.0, less than 5.0, less than 2.5, or less than 2.0.
[74] The method according to any one of items 70 to 73, wherein the polyunsaturated fatty acid and/or the derivative thereof satisfies at least one of the following conditions:
   (1) a ratio of a content of stearic acid and/or a derivative thereof to a content of the polyunsaturated fatty acid having 30 carbons and/or the derivative thereof is less than 50, less than 30, less than 20, or less than 10;
   (2) a ratio of a content of stearic acid and/or a derivative thereof to a content of the polyunsaturated fatty acid having 32 carbons and/or the derivative thereof is less than 15, less than 10, or less than 5;
   (3) a ratio of a content of stearic acid and/or a derivative thereof to a content of the polyunsaturated fatty acid having 34 carbons and/or the derivative thereof is less than 10, less than 5, or less than 2; and
   (4) a ratio of a content of stearic acid and/or a derivative thereof to a content of the polyunsaturated fatty acid having 36 carbons and/or the derivative thereof is less than 20.
[75] The method according to any one of items 70 to 74, wherein the polyunsaturated fatty acid and/or the derivative thereof comprises an impurity having a chemical formula of C23H36O3.
[76] Microbial oil produced by the method according to any one of items 62 to 75.

### Advantageous Effects of Invention

Provided are producing of high concentrations of very long chain polyunsaturated fatty acids and/or derivatives thereof and compositions that comprise very long chain polyunsaturated fatty acids and/or the derivatives thereof.

### Description of Embodiments

### (1) Polyunsaturated Fatty Acids (PUFAs)

In this disclosure, polyunsaturated fatty acids refer to unsaturated fatty acids having two or more valences, preferably three or more valences. The number of double bonds is not limited, but is 2 to 12. Polyunsaturated fatty acids may be n-3 (ω-3), n-6 (ω-6), n-7 (ω-7), or n-9 (ω-9) unsaturated fatty acids. From the viewpoint of physiological effects, the polyunsaturated fatty acids may be n-3 (ω-3) or n-6 (ω-6) unsaturated fatty acids. In this disclosure, polyunsaturated fatty acids having from 20 to 29 carbons are referred to as "PUFA" unless otherwise specified, and polyunsaturated fatty acids having at least 30 carbons are referred to as very long chain polyunsaturated fatty acids (VLC-PUFA). The number of carbons of the polyunsaturated fatty acid refers to the number of carbons of the constituent fatty acids.

Examples of the polyunsaturated fatty acids having from 20 to 29 carbons include polyunsaturated fatty acids having from 20 to 22 carbons, specifically, eicosadienoic acid (C20:2, n-9, EDA), dihomo-γ-linolenic acid (C20:3, n-6, DGLA), mead acid (C20:3, n-9, MA), eicosatetraenoic acid (C20:4, n-3, ETA), arachidonic acid (C20:4, n-6, ARA), eicosapentaenoic acid (C20:5, n-3, EPA), docosatetraenoic acid (C22:4, n-6, ETA), docosapentaenoic acid (C22:5, n-3, n-3DPA), docosapentaenoic acid (C22:5, n-6, n-6DPA), and docosahexaenoic acid (C22:6, n-3, DHA). VLC-PUFAs can be prepared by using these PUFAs as substrates.

### (2) Very Long Chain Polyunsaturated Fatty Acids (VLC-PUFAs)

In this disclosure, "very long chain polyunsaturated fatty acids (VLC-PUFAs)" refers to polyunsaturated fatty acids having at least 30 carbons. An upper limit of the number of carbons is not limited, but is less than 40 or less than 38 from the viewpoint of production by microorganisms. By using polyunsaturated fatty acids having odd numbers of carbons as substrates, polyunsaturated fatty acids having 31, 33, 35, and the like carbons can also be produced.

In this disclosure, the polyunsaturated fatty acids having at least 30 carbons comprise at least one polyunsaturated fatty acid selected from the group consisting of the following:
(1) polyunsaturated fatty acids having 30 carbons;
(2) polyunsaturated fatty acids having 32 carbons;
(3) polyunsaturated fatty acids having 34 carbons; and
(4) polyunsaturated fatty acids having 36 carbons. As an example, the polyunsaturated fatty acids having at least 30 carbons comprise all polyunsaturated fatty acids of (1) to (4).

In this disclosure, the fatty acids refer to free fatty acids. Examples of derivatives of the free fatty acids include triglycerides, diglycerides, monoglycerides, phospholipids, glycolipids, and acylcholesterols. The crude oil, microbial oil, or composition of this disclosure comprises the free fatty acids and the derivatives thereof, and a content of monoglycerides, diglycerides, triglycerides, phospholipids, glycolipids, and acylcholesterols is at least 70 wt.%, at least 80 wt.%, or at least 90 wt.%. When a content of triglycerides in the crude oil, microbial oil, or composition of this disclosure is at least 70 wt.%, hygroscopicity is not too low, so good fluidity can be obtained, for example. Although an upper limit of the content of triglycerides in the crude oil, microbial oil, or composition is not limited, generally a weight ratio of triglycerides in the microbial oil is 99 wt.% or less. The weight ratio of triglycerides in the microbial oil may be 100 wt.%, that is, the microbial oil may be substantially free of components other than triglycerides. In the crude oil, microbial oil, or composition of this disclosure, at least one fatty acid among the constituent fatty acids of the triglyceride is a polyunsaturated fatty acid of any of (1) to (4). In the crude oil, microbial oil, or composition of this disclosure, at least one fatty acid among the constituent fatty acids of the triglyceride is a polyunsaturated fatty acid of any of (1) to (4), and among the constituent fatty acids of the triglyceride, a fatty acid other than the polyunsaturated fatty acids of (1) to (4) is a fatty acid having from 8 to 22 hydrocarbons.

The crude oil, microbial oil, or composition of this disclosure may comprise PUFAs used as substrates. A content of the PUFAs used as the substrates is not limited as long as the PUFAs are present, and is, as an example, at least 0.5 area%, at least 1.0 area%, at least 5.0 area%, at least 10 area%, at least 15 area%, at least 20 area%, or at least 25 area%. An upper limit of the content of the PUFAs used as the substrates is not limited, and the content of the PUFAs used as the substrate is, as an example, 60 area% or less, 50 area% or less, or 40 area% or less.

In the crude oil, microbial oil, or composition of this disclosure, a content of phospholipids is 10 wt.%, or less, 5 wt.%, or less, or 1 wt.% or less of the total weight. However, some phospholipids may be present, for example, 0.1 to 10 wt.%, 0.5 to 7 wt.%, or 1 to 5 wt.% of the total weight of the microbial oil. The phospholipids are removed during a refining process. Thus, refined oils are substantially free of phospholipids.

In the composition, crude oil, or microbial oil of this disclosure, a content of VLC-PUFAs is at least 1.0 area%, at least 1.5 area%, at least 2.0 area%, or at least 2.5 area% as constituent fatty acids of the oil.

Examples of specific host-derived constituent fatty acids comprised in the composition, crude oil, or microbial oil of this disclosure include palmitic acids (C16:0), stearic acids (C18:0), C16:1 fatty acids, and C18:1 fatty acids. In this disclosure, from the viewpoint of increasing a production rate of very long chain polyunsaturated fatty acids, a ratio of specific host-derived constituent fatty acids to the very long chain polyunsaturated fatty acids produced is preferably reduced. In addition, saturated fatty acids are associated with risk of cardiovascular disease and increase a melting point of the oil, so a reduction in a content of palmitic acid and stearic acid is desired.

In the composition, crude oil, or microbial oil of this disclosure, a ratio of the content of stearic acid and/or derivatives thereof to the total content of the very long chain polyunsaturated fatty acids having at least 30 carbons and/or the derivatives thereof is less than 5.0, less than 2.5, or less than 2.0. In addition, the composition, crude oil, or microbial oil of this disclosure satisfies at least one of the following conditions:
(1) a ratio of a content of stearic acid and/or derivatives thereof to a content of the polyunsaturated fatty acids having 30 carbons and/or the derivatives thereof is less than 50, less than 30, less than 20, or less than 10;
(2) a ratio of a content of stearic acid and/or derivatives thereof to a content of the polyunsaturated fatty acids having 32 carbons and/or the derivatives thereof is less than 15, less than 10, or less than 5;
(3) a ratio of a content of stearic acid and/or derivatives thereof to a content of the polyunsaturated fatty acids having 34 carbons and/or the derivatives thereof is less than 10, less than 5, or less than 2; and
(4) a ratio of a content of stearic acid and/or derivatives thereof to a content of the polyunsaturated fatty acids having 36 carbons and/or the derivatives thereof is less than 20.

Composition analysis of fatty acids for the composition, crude oil, or microbial oil of this disclosure by gas chromatography-mass spectrometry revealed that they comprise an impurity having a composition formula of C23H36O3. Thus, the impurity having a composition formula of C23H36O3 help identify the microbial oil of this disclosure. On the other hand, from the viewpoint of increasing the content of very long chain polyunsaturated fatty acids, a ratio of the impurity having a composition formula of C23H36O3 to the very long chain polyunsaturated fatty acids produced is preferably reduced. C23H36O3 may be identified by any technique, and is identified by, as an example, gas chromatography and/or mass spectrometry described in Example. In this case, a substance in a fraction with an APGC retention time of 18.034 minutes can be identified as an impurity, and a substance having a presumed [M+H]⁺ of 361.2710 can be identified as the impurity.

In the composition, crude oil, or microbial oil of this disclosure, a ratio of a content of the impurity having a composition formula of C23H36O3 to the total content of the very long chain polyunsaturated fatty acids having at least 30 carbons and/or the derivatives thereof is less than 0.17, less than 0.16, less than 0.12, or less than 0.10. In addition, the composition, crude oil, or microbial oil of this disclosure satisfies at least one of the following conditions:
(1) a ratio of the content of the impurity having a composition formula of C23H36O3 to a content of the polyunsaturated fatty acids having 30 carbons and/or the derivatives thereof is less than 15, less than 5, less than 3, or less than 1.5;
(2) a ratio of the content of the impurity having a composition formula of C23H36O3 to a content of the polyunsaturated fatty acids having 32 carbons and/or the derivatives thereof is less than 0.3; and
(3) a ratio of the content of the impurity having a composition formula of C23H36O3 to a content of the polyunsaturated fatty acids having 34 carbons and/or the derivatives thereof is less than 0.35, less than 0.3, or less than 0.2.

In the composition, crude oil, or microbial oil of this disclosure, a ratio of a content of specific host-derived constituent fatty acids to the content of very long chain polyunsaturated fatty acids is expressed as "(content of specific host-derived constituent fatty acids)/(content of VLC-PUFAs)". A ratio of the content of the composition formula of C23H36O3 to the content of very long chain polyunsaturated fatty acids is expressed as "(content of the impurity having the composition formula of C23H36O3)/(content of VLC-PUFAs)". These ratios are ratios of contents obtained by analyzing fatty acid composition comprised in the composition, crude oil, or microbial oil.

The fatty acid composition may be determined by the known fatty acid analysis. As an example, an oil to be analyzed is esterified with a lower alcohol and a catalyst to obtain fatty acid lower alcohol esters. Thereafter, the obtained fatty acid lower alcohol esters are analyzed using gas chromatography. Peaks corresponding to the respective fatty acids can be identified in a gas chromatogram obtained, and peak areas of the respective fatty acids can be determined using, for example, the Agilent ChemStation integration algorithm (revision C.01.03[37], Agilent Technologies).

"Area%" used in the fatty acid composition in this disclosure refers to a ratio (percentage) of a peak area of each component to a total peak area in a chromatogram obtained by fatty acid analysis. As an example, "area%" refers to a content of each constituent component at a peak determined by an analysis chart obtained by analyzing the oil having various fatty acids by gas chromatography or (thin layer chromatography)/(flame ionization detector) (TLC/FID). The fatty acid composition was determined by gas chromatography, for example, according to the method indicated in Example. Lipid composition was determined by TLC/FID. Detailed conditions are indicated in Examples.

For convenience, the term "fatty acid" may refer not only to free saturated or unsaturated fatty acids themselves, but also to fatty acids as constituent units comprised in alkyl esters, triglycerides, diglycerides, monoglycerides, phospholipids, steryl esters, and the like, and can also be called constituent fatty acids. In this disclosure, unless otherwise noted, forms of compounds comprising fatty acids may be omitted. Examples of forms of compounds comprising fatty acids include a free fatty acid form, a fatty acid alkyl ester form, a glyceryl ester form, a phospholipid form, and a steryl ester form. Compounds comprising the same fatty acids may be comprised in a single form or may be comprised as a mixture of two or more forms in the oil.

For denoting fatty acids, a numerical expression may be used, wherein the number of carbons, the number of double bonds, and the locations of the double bonds are expressed in a simplified manner using numbers and alphabets, respectively. For example, a saturated fatty acid having 20 carbons is expressed as "C20:0", and a trivalent unsaturated fatty acid having 20 carbons and having three double bonds in the carbon chain is expressed as "C20:3". For example, behenic acid may be expressed as "C22:0", arachidonic acid as "C20:4, n-6", and the like. "n-" indicates a position of the first double bond counted from a methyl end of the fatty acid. For example, "n-6" indicates that the position of the double bond is the sixth position counted from the methyl end of the fatty acid, and "n-3" indicates that the position of the double bond is the third position from the methyl end of the fatty acid. This method is known to those of ordinary skill in the art, and those of ordinary skill in the art can easily specify fatty acids expressed in accordance with this method.

### (3) Composition

"Composition" of this disclosure is a lipid composition comprising very long chain polyunsaturated fatty acids and/or derivatives thereof. In one example, the composition of this disclosure is characterized by further comprising cells that produce the very long chain polyunsaturated fatty acids and/or the derivatives thereof and/or crushed materials of the cells.

In another example, the composition of this disclosure is characterized by a content of the very long chain polyunsaturated fatty acids and/or the derivatives thereof and a total content of acylcholesterols, phospholipids, glycolipids, monoglycerides, diglycerides, and triglycerides. Acylcholesterols, phospholipids, glycolipids, monoglycerides, diglycerides, and triglycerides are usually removed during concentration to increase the concentration of the very long chain polyunsaturated fatty acids and/or the derivatives thereof.

The composition of this disclosure comprises high concentrations of the very long chain polyunsaturated fatty acids and/or the derivatives thereof, for example, the very long chain polyunsaturated fatty acids and/or the derivatives thereof at a concentration of at least 1.0 area%, at least 1.5 area%, at least 2.0 area%, or at least 2.5 area%. Further, in the composition of this disclosure, the total content of acylcholesterols, phospholipids, glycolipids, monoglycerides, diglycerides, and triglycerides is at least 70 wt.%.

Cells comprised in the composition of this disclosure are cells in which a PUFA chain elongase gene is expressed. Such cells produce the very long chain polyunsaturated fatty acids and/or the derivatives thereof comprised in the composition of this disclosure. Such cells may be any of animal cells, plant cells, and microorganisms.

The crushed materials are materials obtained from cells that have been crushed, and include any components such as cell-derived lipids, proteins, sugar chains, and nucleic acids. Thus, it is also possible to detect components of the cells from which the very long chain unsaturated fatty acids and/or the derivatives thereof comprised in the composition are derived. Examples of such components include antigens specific to these cells and nucleic acids specific to these cells. In particular, the transduced PUFA chain elongase gene can be detected by a probe, PCR, or the like. In specific, the presence of the transduced PUFA chain elongase gene can be detected by performing PCR using a predetermined primer set, and in some cases performing nested PCR.

### (4) Microbial oil

In this disclosure, the term "microbial oil" broadly refers to all lipids obtained from microorganisms, unless otherwise noted, and is used in this disclosure as a term to refer to both crude oil and refined oil without distinction. The microbial oil is obtained by culturing microorganisms in an appropriate culture medium and collecting microbial cells by a method such as solvent extraction. Even when the microbial oil is a refined oil, the microbial oil is only refined until some components with microbial characteristics remain, and may be distinguished from concentrates of specific oils and fats. The microbial oil of this disclosure is characterized by the content of very long chain polyunsaturated fatty acids (VLC-PUFA). In addition, the microbial oil of this disclosure is further characterized by a ratio of the content of specific host-derived constituent fatty acids to a content of VLC-PUFA comprised in the microbial oil. The reduced ratio of the content of specific host-derived constituent fatty acids to the content of VLC-PUFAs reduces influence of the host-derived fatty acids, and risk of comprising host-derived impurities when refining VLC-PUFAs from the microbial oil. The low ratio of the content of specific host-derived constituent fatty acids to the content of VLC-PUFAs can reduce a lipid dosage when administering VLC-PUFAs as an active ingredient.

The microbial oil in this disclosure refers to an oil obtained from any microorganism. The microbial oil can also be referred to as yeast oil, fungal oil, or algal oil depending on a type of microorganism. In addition, instead of microorganisms, cells can be used to obtain oil, which can also be referred to as cell oil.

"Crude oil" refers to oil in a state of being pressed or extracted from oil-bearing raw materials. In this disclosure, a mixture of lipids obtained by simply extracting lipids from microbial cells is referred to as "crude oil" of microbial oil. Impurities such as phospholipids and cholesterol are removed from crude oil to obtain a refined oil. Since crude oil is oil and fat before refining, the fatty acid composition thereof depends on the fatty acid composition in the oil-bearing raw material. The crude oil of this disclosure is characterized by a content of the very long chain polyunsaturated fatty acids and/or the derivatives thereof of at least 1.0 area%, at least 1.5 area%, at least 2.0 area%, or at least 2.5 area%.

"Refined oil" refers to an oil obtained after removing substances other than the target substances, such as phospholipids and sterols, through a refining process that combines some or all of de-gumming process, deoxidation process, de-coloring process, deodorization process, and the like. Through the refining process, a ratio of triglycerides is increased.

The term "oil" is generally defined as a liquid in water-immiscible combustible substances that phase separates from water. "Fat" is defined as a solid in water-immiscible combustible substances that phase separates from water, and both are collectively referred to as "oil and fat". The term "lipid" is generally defined as a substance of biological origin that is insoluble in water. Customarily, animal oils and fats are referred to as "fats", and vegetable oils and fats or fish-derived oils and fats are referred to as "oils", but the composition determines whether it is solid or liquid, so even vegetable oils and fish oils exist as solids. Thus, in this disclosure, the term "oil" does not necessarily mean a liquid, and may be used interchangeably with "oil and fat" or "lipid" for convenience.

Although the terms "oil" and "oil and fat" mean triglycerides in a narrow sense, in this disclosure, these terms include triglycerides as well as other lipid components such as diglycerides, monoglycerides, phospholipids, glycolipids, cholesterol, and free fatty acids.

### (5) Microorganism

In this disclosure, examples of "microorganisms" include eubacteria, archaea, and eukaryotes. Examples of eukaryotes include algae, protists, fungi, and slime molds. As an example of fungi, ascomycetes such as yeast and filamentous fungi such as molds may be used. As an example of this disclosure, yeast is used. In another example, cells may be used instead of microorganisms. Mammalian cells, plant cells, insect cells, and the like can be used as cells that produce VLC-PUFAs. From the viewpoint of producing VLC-PUFAs, microorganisms or cells gene-transduced with a PUFA chain elongase gene are used.

"Chain elongase" refers to an enzyme having activity of elongating a chain length of fatty acids. Various enzymes are known as chain elongases, and one example thereof is an enzyme belonging to the ELOVL family. From the viewpoint of producing VLC-PUFAs, it is preferable to use PUFA chain elongases having PUFA chain elongation activity using PUFAs as substrates. Among ELOVL4, ELOVL4 that uses PUFAs as substrates can be used as a PUFA chain elongase. Such ELOVL4 that uses PUFAs as substrates may also include enzymes that cannot produce VLC-PUFAs of at least C30. Thus, from the viewpoint of producing VLC-PUFAs, a chain elongase capable of producing VLC-PUFAs of at least C30, such as ELOVL4 capable of producing VLC-PUFAs having at least C30, is preferred. Fish ELOVL4 includes ELOVL4a and ELOVL4b, either of which may be used.

Any enzyme may be used as such an enzyme, and as one example, the following may be used:
a) specific ELOVL4; or
b) a protein comprising an amino acid sequence having at least 40%, 50%, 60%, 70%, 80%, 85%, 90%, 95%, 97%, 98%, or 99% sequence identity to an amino acid sequence encoding the specific ELOVL4, comprising a consensus sequence for chain elongases, and having PUFA chain elongation activity. Examples of consensus sequences for chain elongases include histidine cluster motifs (amino acid sequence: HXXHH, where X represents any amino acid) and/or dilysine motifs (amino acid sequence: XKXKXX or KKXX, where X represents any amino acid) (Mol Vis. 2003 Jul 3; 9:301-307.).

The PUFA chain elongase gene is operably linked downstream of an expression promoter and gene-transduced into the host. Such an expression promoter may be any expression promoter that is expressed in the host. The expression promoter may be either an inducible expression promoter or a constitutive expression promoter. From the viewpoint of promoting the expression of the PUFA chain elongase at a predetermined timing, the inducible expression promoter is used. As the inducible expression promoter, any inducible expression promoter can be used, and as an example, a galactose-inducible promoter can be used. When the PUFA chain elongase gene operably linked under control of a galactose-inducible promoter is transduced into a microorganism, the PUFA chain elongase gene is expressed upon addition of an inducer such as galactose or an analog thereof. As an example of the PUFA chain elongase gene, the following can be used:
a) a polynucleotide encoding specific ELOVL4; or
b) a polynucleotide having a nucleotide sequence having at least 40%, 50%, 60%, 70%, 80%, 85%, 90%, 95%, 97%, 98%, or 99% sequence identity to the polynucleotide encoding the specific ELOVL4 and encoding a protein having PUFA chain elongation activity. By transducing these polynucleotides, VLC-PUFAs of at least C30 can be produced.

The specific ELOVL4 may be ELOVL4 from any species. From the viewpoint of increasing an amount of VLC-PUFAs produced, non-mammalian ELOVL4 is used. Examples of non-mammalian ELOVL4 include avian, fish, reptile, and amphibian ELOVL4, especially fish ELOVL4. Fish ELOVL4 includes ELOVL4a and ELOVL4b, either of which may be used. As an example of fish ELOVL4, ELOVL4b of black seabream or ELOVL4a of African sharptooth catfish can be used. ELOVL4b of black seabream has an amino acid sequence set forth in SEQ ID NO: 11. SEQ ID NO: 12 corresponds to a polynucleotide encoding such an amino acid sequence. ELOVL4a of African sharptooth catfish has an amino acid sequence set forth in SEQ ID NO: 13. SEQ ID NO: 14 corresponds to a polynucleotide encoding such an amino acid sequence.

**[Table 1-1]**

| Table 1. ELOVL4b of black seabream | |
|---|---|
| Accession No . ANJ04909 | MEVVTHFVNDTVEFYKWSLTIADKRVEKWPMMSSPLPTLAISCLYLLFLWAGPRYMQDRQPYTLRKT LIVYNFSMVVLNFYIAKELLLASRAAGYSYLCQPVKYSNDVNEVRIASALWWYYISKGVEFLDTVFF ILRKKFNQVSFLHVYHHCTMFILWWIGIKWVPGGQSFFGATINSSIHVLMYGYYGLAALGPQMQKYL WWKKYLTIIQMIQFHVTIGHAGHSLYTGCPFPAWMQWALIGYAVTFIILFANFYYHAYRGKPSSTQK GGKPVANGTSAVTNGHSKAEEVEENGKRQKKGRAKRE (SEQ ID NO: 11) |

**[Table 1-2]**

| | |
|---|---|
| Accession No . KU372150 | acatggggag tggcacctgg cttccctcgg tgtctaggag attgcaagaa gatgatgcgg ctgagcggac tgaatgcata attggcctat cttagtcata gattttcact tgccttgtcc agtttcttgc caaattgctg tttaatgtcg aactgtagcc ggcgcggtgt agaaacttga ttagcggtga cggcaggaga agggaagaag gggagaagaa ggagaagagg agcggagcgg tgactgagag aggagttggg catcaaattg caccggatgt cattacacgc tcttaaatat |
| | cgaggactat aatcgagacc aaaggcagag ccatggaggt tgtaacacat tttgtgaatg acactgtaga attttacaaa tggagcctta ctatagcaga caagagggtg gagaagtggc ccatgatgtc atctcccctc cccactctgg ccatcagctg cctgtacctg ctcttcctgt gggcggggcc ccgatacatg caggaccgcc agccctacac gctcaggaag accctcatag tctacaactt cagcatggtg gtcctcaatt tctacatcgc caaagagctc ctactagcct ctagagcagc tgggtacagc tacctctgtc aacctgtcaa gtactccaat gatgtcaacg aagtcaggat agcatctgct ctctggtggt actacatctc caaaggcgtg gagttcctgg atacagtctt tttcatcctc aggaagaagt tcaaccaggt cagcttcctc cacgtctacc accactgcac catgttcatc ctctggtgga tcggcatcaa atgggtcccc ggtggacagt cattttttgg tgcaaccatc aactcttcca tccacgtgct catgtacgga tactacggcc tggcagccct gggacctcag atgcagaagt acctctggtg gaagaaatac ctcaccatta tccagatgat ccagttccac gtgaccatcg gccacgccgg ccactccctc tacacaggct gcccattccc cgcctggatg cagtgggctt tgatcggcta cgctgtcacc ttcatcatcc tcttcgccaa cttctactac cacgcttacc gaggcaaacc ttcatccacg cagaagggag gcaagcccgt cgccaacggc acctctgcgg taactaacgg tcacagcaaa gcggaggagg tggaggaaaa tgggaagagg caaaagaagg gaagagcgaa aagggagtaa agaagaaaga gggcgtgctt ggcgattaac agagagagga cagttgtttt aaaaagggcc agagatgaag ggaggagagg gataaacagc gaagttcaaa ccagaccggc ttcattatcc ctgcaaataa tgtagaagga ggtgatgttg gtgaccaact tggaaaggtg gaaaagtagc agtgtgtgtg tttgtggatg tgtctgtaag ggtttctgga tttgaaaggt tgtgttggtt gtcttccata gcagtttttt ttttcttttt taaatcaaga aaaagcaaaa tgctgaccac gtaggatccg cgctcatctg tccaactggg gttttggact gtagtaggac caaagtattt aaaatgggcc tacaagctgc tcagcatcca ttaacttatc aaccaaaatt cactcaactt ttgtactctg gattccagta ttattttaac tgaaagcttg aatagtcttt aatatgctta tttattttct agtgctctaa aagagtttta atgaatttaa ctattaccaa caaaagaaca tgcagaagtt tataccaaag gtttattttc ttggttcggg ttttttttct tgttttacga atatttagca agttagcaaa caaacaaaaa aaggttacaa atctcacgac tgccatctat tgtattgaac tactttttaa ttattcacac tatctaccaa tccaacatgt catggacttt acatttctct gctgtctcaa tctgaagtaa ctgtgctttt ttgcacttgc agacattctc tttgtgttca ttgtgaatta aaatgttggt ttaaaacact ttgggagggg actgtagttt tattttagtc tttatgcaaa tgtgtgggta tatttgttat gatttctttt tgtgggtcct catgatttat ttctttttgc tgtgcgatgt agccgaggtg caagaataag agattgaatt tcagctcagt actgtctcac tgccatgtag gactgaaggg aaacctgaca agagcttttt ttttatttta aagaaaacaa atggttattt cattgtctta ttaagaatca ataaacgaca cagcttcggg ctgcacatat aactgaaata aagacaatgt cagcccccca aaaaaaaaaa aaaaaaaaaa (SEQ ID NO: 12) |

**[Table 2]**

| Table 2. ELOVL4a of African sharptooth catfish | |
|---|---|
| Accession No. ASY01350 | MDIVTHLVNDTIEFYKWSLTIADKRVEKWPLMGSPLPTLAISSSYLLFLWLGPKF MRNREAFQLRKTLIVYNFSMVILNFFIFKELFLAARAANYSYLCQPVDYSDDPNE VRVAAALWWYFVSKGVEYLDTVFFILRKKFNHVSFLHVYHHCTMFTLWWIGIKW VAGGQSFFGAHMNAAIHVLMYLYYGLAACGPKIQKYLWWKKYLTIIQMIQFHVTI GHTALSLYTDCPFPKWMHWCLIGYALTFIVLFGNFYYQTYRRQPRREGLSKAGK ALSNGASNGMAISNGVSGKMVEKPVVVENGRRKRKGRAKRD (SEQ ID NO: 13) |
| Accession No. KY801284 | aaactgtttc cactggagcg ccgagagagc agcagccaca aacgcactga tgcaaatgtc cgtgtaggat gctctgtaca tacacccagg acaattagga cgtattttgc agagatggat attgtaacac atttagtcaa cgacactatc gaattctaca aatggagtct taccattgca gacaaacgtg tggaaaaatg gcctcttatg ggctctcctc tccccacact ggccatcagc tcctcatacc tgctcttcct ctggctgggg ccaaagttca tgcggaacag ggaggctttt cagctcagga agaccctaat tgtgtacaat ttcagcatgg ttattcttaa ctttttcatc ttcaaagagc tcttccttgc agcaagggca gcaaactaca gctacctctg ccagccagtg gactattctg atgaccctaa tgaagtcagg gttgcagctg ctctctggtg gtactttgtg tcaaaggggg ttgaatatct ggacacagtg tttttcatcc ttcgtaaaaa gttcaaccat gtcagctttt tgcacgtgta tcaccactgc accatgttta cactgtggtg gattggcatc aaatgggttg caggtggaca gtcatttttt ggtgctcata tgaatgctgc aatccatgtt cttatgtatc tatattatgg actggctgcg tgtggtccca agatccagaa atacttgtgg tggaagaagt acctgacaat tatccaaatg atccagttcc atgtcactat tggtcatact gctctatcgc tctacactga ctgccccttc cctaaatgga tgcactggtg tctgattggt tatgccctca ccttcattgt cctctttggg aacttttact accagacata tcgtcgccag ccacgccgtg aaggtctgtc caaggctggc aaggcactgt ctaatggagc ttctaatgga atggcaatca gtaatggagt gagtggcaag atggtggaga agcctgtggt agtggaaaat gggaggagga agaggaaggg cagggcaaag cgggactaga gcagatgaag gattgagatg cagaagcagg catatttgag aaaggatata agagatggga catttgttaa ggagcagtac atttgtagga tgccagggat gaggatatga ctgtttggag gaggctcaaa ggtcactgtg tgggctttgc taccaccagt tagggtggat aagattagaa atatttgtta agaacaaaca tttggtcctt ctgtttacag ctgtgttttt gctgatttta tgcataagca accctgtaat aagaatgaaa tagtcatgat taagggagcc tgaattctct atcaaagcat acaattattt aaacaattcc taaaaaaaaa aaa |
| | (SEQ ID NO: 14) |

### (6) Production of VLC-PUFAs and/or derivatives thereof

### (6-1) Production Step

VLC-PUFAs and/or derivatives thereof can be produced by a production method of this disclosure. The manufacturing method of this disclosure is characterized by comprising the following steps:
(A) transducing an expression system for a PUFA chain elongase into cells,
(B) conditioning the transduced cells to a state of high VLC-PUFA content, and
(C) inducing expression of the PUFA chain elongase in the cells conditioned. The VLC-PUFAs and/or the derivatives thereof are produced by such a production method and accumulate in a culture. Through the steps in the manufacturing method described above, a culture comprising a high content of the VLC-PUFAs and/or the derivatives thereof can be obtained.

"Culture" means anything obtained by culturing, including cell oils, cell bodies, and a culture medium. "Culture comprising cell bodies" means, in particular, a culture wherein "cells" are cultured, before the cells are separated from the culture medium. Cells comprising cell oil can be obtained from the culture. The cells may include both living and dead cells. Dried cells are also included. The expression "cells" refers to dried cells that are substantially free of water and dried cells that comprise residual culture medium components, filter aids, and the like. "Substantially free of water" means a water content below which cell survival may be difficult. This water content is generally 15 wt.% or less, or 10 wt.% or less. According to this disclosure, cell oils and/or cells comprising VLC-PUFAs are provided. Among such cell oils and/or cell bodies, cell oils and/or cell bodies comprising at least 1.0 area% of the polyunsaturated fatty acids having at least 30 carbons and/or the derivatives thereof have not been known. Thus, use of the cell oils of this disclosure and the cell bodies of this disclosure can efficiently provide oils comprising VLC-PUFAs at higher concentrations than existing oils.

The step of transducing an expression system for a PUFA chain elongase into cells can be carried out by transducing a vector comprising a PUFA chain elongase gene operably linked downstream of an expression promoter into cells according to a standard method. A type of and a transduction method of vector can be appropriately selected for the host cell. Alternatively, the expression system for a PUFA chain elongase may be transduced by genome editing. By modifying a gene encoding an existing PUFA chain elongase, genome editing may be performed so that a chain elongase capable of producing VLC-PUFAs of at least C30 is expressed, or a chain elongase capable of producing VLC-PUFAs may be newly incorporated into the genome.

The step of conditioning the transduced cells to a state of high VLC-PUFA content includes a step of culturing the transduced cells, and may further include a step of conditioning turbidity and/or a step of adding substrates, to induce expression of the PUFA chain elongase gene so that the VLC-PUFA content is high. The culture of the transduced cells may be aeration and agitation culture, shaking culture, or static culture, and can be appropriately selected depending on the cells to be cultured. As an example, when yeast is used, culture with aeration and agitation usually requires one to seven days. The aeration rate during aeration and agitation culture may be a normally used aeration rate for such aeration. Any culture medium known in this technical field may be used for a culture medium to be used in the culture, depending on the cells to be used.

For a liquid culture medium, any of commonly used carbon sources can be used, including but not limited to glucose, fructose, xylose, saccharose, maltose, soluble starch, molasses, glycerol, and mannitol.

Examples of nitrogen sources include natural nitrogen sources such as peptone, yeast extract, malt extract, meat extract, casamino acid, and corn steep liquor, organic nitrogen sources such as urea, and inorganic nitrogen sources such as sodium nitrate, ammonium nitrate, and ammonium sulfate. In addition, inorganic salts such as phosphates, magnesium sulfate, iron sulfate, copper sulfate as well as vitamins and the like can also be used as micronutrient sources as needed. An aqueous medium that can be used as a base material of the liquid culture medium is basically water, and may be distilled water or purified water.

In order to promote VLC-PUFA productivity, substrates for VLC-PUFAs can be added to the culture medium. PUFAs can be added as the substrates for VLC-PUFAs. The PUFAs to be added as the substrates for VLC-PUFAs can be appropriately selected according to the desired VLC-PUFAs. Examples of such PUFAs include, but are not limited to, eicosapentaenoic acid (C20:5, n-3), docosapentaenoic acid (C22:5, n-3), docosahexaenoic acid (C20:6, n-3), arachidonic acid (C20:4, n-6), and docosapentaenoic acid (C22:5, n-6). PUFAs can be added to the culture medium at a concentration of 0.01 to 5% as an example. From the viewpoint of not reducing growth of microorganisms, 3% or 1% can be used as an upper limit of the PUFA concentration. From the viewpoint of increasing a production amount of VLC-PUFAs, 0.05% or 0.1% can be used as a lower limit of the PUFA concentration. The PUFAs may be comprised in the culture medium from the beginning, or may be added at the timing when the expression-inducing treatment for the PUFA chain elongase gene is performed. Hosts capable of producing PUFAs are limited. For example, yeast cannot produce PUFAs without artificial manipulation such as transduction of foreign genes or genome editing. Such hosts can be referred to as non-PUFA-producing hosts. When a non-PUFA-producing host is used, all PUFAs comprised in the culture are derived from the added substrate.

A culture vessel used in the production step is not limited, and any culture vessel that is normally used for culturing microorganisms can be used. The culture vessel can be appropriately selected according to a scale of culture.

The step of inducing expression of the PUFA chain elongase in the cells conditioned includes performing an induction treatment at a predetermined timing according to the expression system of the PUFA chain elongase. When a galactose-inducible promoter is used as the expression system, the PUFA chain elongase is expressed by adding galactose or an analog thereof to the culture medium. The induction treatment is carried out during a stationary phase of the culture or during a period when cell proliferation is stagnant due to stress. The stationary phase is brought about by depletion of carbon sources such as sugar, and can be said to be in a stress state due to nutrient starvation. In addition to nutrient starvation stress, temperature condition stress, light condition stress, and the like can also cause cell proliferation to stagnate. Examples of the temperature condition stress include low temperature stress and high temperature stress (heat shock). Examples of the light condition stress include ultraviolet irradiation and lack of light having a specific wavelength. An example of the stationary phase is when the microorganisms are cultured and a turbidity value reaches 1.5 to 11. The turbidity value can be measured using a spectrophotometer, and in Example, a double beam spectrophotometer (U2910, manufactured by Hitachi Ltd.) was used. When the turbidity value reaches 1.5 to 11, the elovl4 gene is induced to increase the content of VLC-PUFAs produced. While not intending to be limited by theory, possible reasons for the production of high concentrations of VLC-PUFAs during a period of stress-induced stagnation of cell proliferation include the following:
(1) fatty acids serving as substrates for VLC-PUFAs are increased, that is, C16 and C18, which are required in large amounts during cell proliferation, can be supplied in large amounts due to stagnation of cell proliferation;
(2) any of activity, stability, or localization of the PUFA chain elongases, or a combination thereof, is in a state that contributes to higher production of VLC-PUFAs with the stationary phase in culture or stagnation of the cell proliferation, and/or
(3) degradation of VLC-PUFAs themselves is in a state of decreasing with the stationary phase in culture or the stagnation of cell proliferation.

### (6-2) Separation Step

In a separation step, the cell oil comprising VLC-PUFAs produced during the production step is separated from the culture. The separation step includes separating the cultured cells from the culture medium used for culture (hereinafter referred to as a cell separation step) and collecting the cell oil comprising VLC-PUFAs from the cultured cells (hereinafter cell oil collection step), that is, obtaining the crude oil. In the cell separation step and the cell oil collection step, cell oil comprising VLC-PUFAs is collected from the cells using a separation method and an extraction method according to the culture form.

When a liquid culture medium is used, cell bodies can be separated from the culture medium after the culture is completed by normal solid-liquid separation means such as centrifugation and/or filtration. The cell bodies are thoroughly washed with water and then dried, as needed. Drying may be performed by freeze drying, air-drying, heating-drying, or the like.
When cultured in a solid culture medium, the solid culture medium and cell bodies may be crushed using a homogenizer or the like without separating the cell bodies from the culture medium, and the resulting crushed material may be directly subjected to the cell oil collection step.

The cell oil collection step may include extracting the dried cells obtained in the cell separation step by using an organic solvent under a nitrogen gas stream. Examples of organic solvents that can be used include ether, hexane, methanol, ethanol, chloroform, dichloromethane, and petroleum ether. Alternatively, good results can be obtained by alternate extraction with methanol and petroleum ether, or extraction using a one-phase solvent system of chloroform-methanol-water. Distillation of the organic solvent from the extract under reduced pressure yields a cell oil comprising VLC-PUFAs. Hexane is most generally used when collecting triglycerides. Instead of the aforementioned method, wet cells can be used for extraction. A water-miscible solvent such as methanol or ethanol, or a water miscible mixed solvent comprising this solvent with water and/or other solvents can be used. The rest of the procedure is similar to that described above.

The crude oil of the collected cell oil may be refined by a method used for refining vegetable oil, fish oil, or the like. Examples of refining process normally performed for oils and fats include de-gumming, deoxidation, de-coloring, and deodorization. Any of these treatments may be carried out by any method. A water washing treatment is an example of the de-gumming. A distillation treatment is an example of the deoxidation treatment. Treatments with activated clay, activated carbon, and silica gel are examples of the de-coloring treatment. Steam distillation is an example of the deodorization.

The cell oil, microbial oil, crude oil, or composition comprising VLC-PUFAs can be used, for example, in foods, supplements, pharmaceuticals, cosmetics, and animal feed. This disclosure relates to pharmaceutical compositions, cosmetic compositions, food products, supplements, or animal feeds comprising cell oils, microbial oils, crude oils, or compositions according to this disclosure. Another aspect also relates to cell oils, microbial oils, crude oils, or compositions for use as medicaments. Pharmaceuticals, cosmetics, foods, supplements, or animal feeds of this disclosure may be used for prevention, treatment, or remission of cellular senescence or Stargardt's disease. Yet another aspect relates to a method of treating or preventing cellular senescence or Stargardt's disease, including administering to a subject a cell oil, microbial oil, crude oil, or composition according to this disclosure. This method includes administering a cell oil, microbial oil, crude oil, or composition according to this disclosure to a subject suffering from or at risk of suffering from cellular senescence or Stargardt's disease.

In some cases, the cell oil, microbial oil, crude oil, or composition of this disclosure may be administered with any other therapeutic agent (e.g., corticosteroid) used in the pharmaceutical applications described above.

In this disclosure, the term "step" includes not only an independent step but also a step that cannot be clearly distinguished from other steps as long as an intended purpose of the step is achieved. In this disclosure, a numerical range indicated using "to" is a range including numerical values described before and after the "to" as the minimum and maximum values, respectively. In this disclosure, when the mixture comprises multiple substances corresponding to each component, unless otherwise specified, the content of each component in the mixture means the total content of the multiple substances present in the mixture. In this disclosure, the term "not more than" or "less than" with regard to a percentage means to include 0%, that is, "does not comprise", or include a range including a value undetectable by existing means, unless the lower limit value is specifically stated.

All documents referred to herein are incorporated herein by reference in their entirety.

Examples of the present invention described below are for illustrative purposes only and are not intended to limit the scope of the present invention. The technical scope of the present invention is limited only by the description of the claims. Modifications of the present invention, such as additions, deletions, and substitutions of constituent elements of the present invention, can be made without departing from the spirit of the present invention.

### Examples

### Example 1: Preparation of Mouse-derived ELOVL4 Gene Expression Strain

A mouse *(Mus muscules)-derived* ELOVL4 gene (ACCESSION No. AF277093) (hereinafter referred to as MmELOVL4) with a codon optimized for expression in budding yeast was artificially synthesized. A synthesized DNA fragment was used as a template to amplify an MmELOVL4 gene fragment using a primer pair of SEQ ID NO: 1 and SEQ ID NO: 2 shown in Table 3. The amplified fragments were fused with a pYES2 NT/A vector (Thermo Fisher Scientific Inc.) cleaved with restriction enzymes HindIII and KpnI using the In-Fusion HD Cloning Kit (Takara Bio, Inc.) to construct a plasmid for MmELOV4 gene expression.

This plasmid for expression comprises an insert sequence wherein a Gal1 promoter sequence, the MmELOVL4 gene, and a CYC1 terminator sequence are linked in this order, and a pYES2 NT/A vector sequence comprising a ura3 gene.

The prepared plasmid for MmELOVL4 expression was transduced into a budding yeast *S. cerevisiae* INVSc1 strain (Thermo Fisher Scientific Inc.) by the lithium acetate method, and a gene recombinant was obtained using uracil requirement as an indicator. For comparison, a *S*. *cerevisiae* INVSc1 strain into which a pYES2 NT/A vector was transduced was also obtained.

Transduction by the lithium acetate method was carried out as follows.
The *S. cerevisiae* INVSc1 strain was cultured in 5 ml of YPD medium (Polypepton 20 g/L, Yeast extract 10 g/L, Glucose 20 g/L) at 28°C and 180rpm for 16 hours. 0.5 ml of a culture solution was aseptically collected in a 1.5 ml centrifuge tube, centrifuged at 3000 xg for 3 minutes, and a supernatant was discarded. To a precipitate, 1 µg of the plasmid for MmELOVL4 expression was added together with 100 µl of lithium acetate solution (0.2 M lithium acetate, 40% (w/v) polyethylene glycol 4000, 0.1 M Dithiothreitol) and suspended. The 1.5 ml centrifuge tube comprising a suspension was heated in a water bath at 43.5°C for 45 minutes. Thereafter, an entire amount was applied to SD-ura agar medium (Minimal SD Agar Base (Takara Bio, Inc.) 46.7 g/L, - Ura DO Supplement (Takara Bio, Inc.) 0.77 g/L, purified agar powder (NACALAI TESQUE INC.) 15 g/L, adjusted to pH 5 8)), and cultured statically at 28°C for 2 to 3 days. Colonies formed on the SD-ura agar medium were obtained as candidate strains of gene recombinants, and the presence or absence of insertion of each gene was determined based on the presence or absence of amplification of the target gene by a PCR method.

**[Table 3]**

| Table 3: List of primers used | |
|---|---|
| MmELOVL4 forward primer | AGGGAATATTAAGCTTACCATGGGGCTGCTGGACTCAGA (SEQ ID NO: 1) |
| MmELOVL4 reverse primer | CACACTGGATCCTAGGTACCTTACTCTCCTTTTGGCTTCCCGTTT (SEQ ID NO: 2) |
| AsELOVL4 forward primer | AGGGAATATTAAGCTTACCATGGAGGTTGTAACACATTTTGTGA (SEQ ID NO: 3) |
| AsELOVL4 reverse primer | CACACTGGATCCTAGGTACCCTACTCCCTTTTCGCTCTTCCCT (SEQ ID NO: 4) |
| PGK1promoter forward primer | GATGATCCACTAGTAGGAAGTACCTTCAAAGAATG (SEQ ID NO: 5) |
| PGK1promoter reverse primer | GATCCGGGGTTTTTTTTGTTTTATATTTGTTGTAAAAAGTAG (SEQ ID NO: 6) |
| InversePCR forward primer | AAAAAACCCCGGATCGGACTACTAGCAG (SEQ ID NO: 7) |
| InversePCR reverse primer | TACTAGTGGATCATCCCCACGCGCCCTGT (SEQ ID NO: 8) |
| CgELOVL4 forward primer | AGGGAATATTAAGCTTACCATGGATATTGTAACACATTTAGTCAAC G (SEQ ID NO: 9) |
| CgELOVL4 reverse primer | CACACTGGATCCTAGGTACCCTAGTCCCGCTTTGCCCTGCCCTT (SEQ ID NO: 10) |

### Example 2: Preparation of Black Seabream-derived ELOVL4b Gene Expression Strain

A black seabream *(Acanthopagrus schlegelii*)-derived ELOVL4b gene (ACCESSION No. KU372150) (hereinafter referred to as AsELOVL4b) with a codon optimized for expression in budding yeast was artificially synthesized. A synthesized DNA fragment was used as a template to amplify an AsELOVL4b gene fragment using a primer pair of SEQ ID NO: 3 and SEQ ID NO: 4 shown in Table 3. The amplified fragments were fused with a pYES2 NT/A vector (Thermo Fisher Scientific Inc.) cleaved with restriction enzymes HindIII and KpnI using the In-Fusion HD Cloning Kit (Takara Bio, Inc.) to construct a plasmid for AsELOV4b gene expression.

This plasmid for expression comprises an insert sequence wherein a Gal1 promoter sequence, the AsELOVL4b gene, and a CYC1 terminator sequence are linked in this order, and a pYES2 NT/A vector sequence comprising a ura3 gene.

The prepared plasmid for AsELOVL4b expression was transduced into a budding yeast *S. cerevisiae* INVSc1 strain (Thermo Fisher Scientific Inc.) by the lithium acetate method described in Example 1, and a gene recombinant was obtained using uracil requirement as an indicator.

### Example 3: Preparation of Constitutively Expressing AsELOVL4b Gene Expression Strain

A phosphoglycerate kinase 1 promoter (hereinafter referred to as PGK1 promoter) was artificially synthesized. A synthesized DNA fragment was used as a template to amplify a PGK1 promoter fragment using a primer pair of SEQ ID NO: 5 and SEQ ID NO: 6 shown in Table 3. Further, the plasmid for AsELOVL4b expression prepared in Example 2 was used as a template to amplify a sequence of an AsELOVL4b expression vector other than the Gal1 promoter sequence using a primer pair of SEQ ID NO: 7 and SEQ ID NO: 8 shown in Table 3. Thereafter, the amplified fragments were fused using the In-Fusion HD Cloning Kit (manufactured by Takara Bio, Inc.) to construct a plasmid for constitutively expressing AsELOVL4b gene expression.

This plasmid for expression comprises an insert sequence wherein a PGK1 promoter sequence, the AsELOVL4b gene, and a CYC1 terminator sequence are linked in this order, and a pYES2 NT/A vector sequence comprising a ura3 gene.

The prepared plasmid for constitutively expressing AsELOVL4b expression was transduced into a budding yeast *S. cerevisiae* INVSc1 strain (Thermo Fisher Scientific Inc.) by the lithium acetate method described in Example 1, and a gene recombinant was obtained using uracil requirement as an indicator.

### Example 4: Production of VLC-PUFAs by MmELOVL4 and AsELOVL4b Gene Expression Strains

The MmELOVL4 gene expression strain and the AsELOVL4b gene expression strain prepared by the methods described in Examples 1 and 2, and a pYES NT/A vector-transduced strain were each cultured in 5 ml of SD-ura liquid medium (Minimal SD Agar Base (manufactured by Takara Bio, Inc.) 46.7 g/L, -Ura DO Supplement (manufactured by Takara Bio, Inc.) 0.77 g/L, adjusted to pH 5.8) at 28°C and 180 rpm for 16 hours. 0.1 ml of each culture solution was inoculated into 5 ml of uracil-restricted raffinose liquid medium (Minimal SD Base/Raf (manufactured by Takara Bio, Inc.) 37 g/L, -Ura DO Supplement 0.77 g/L, adjusted to pH 5.8), and cultured until the turbidity value of the culture solution reached 1.0. Thereafter, a 40% galactose solution was added in an amount of 1/20 times the volume of the culture solution, and EPA was further added in an amount of 1/1000 times the volume of the culture medium (5 µl). After addition of the galactose solution and EPA, cells were further cultured for 2 to 5 days, and the entire culture solution was aseptically collected in a 15 ml centrifuge tube and centrifuged at 3000 xg for 3 minutes to collect the cells. Thereafter, the cells were frozen at -80°C and then subjected to a lyophilizer (VA-140S manufactured by TAITEC CORPORATION) to obtain lyophilized cells. A portion of the obtained lyophilized cells was used as a sample for measuring a fatty acid composition.

Total lipids, that is, microbial oils A, B, and C, were obtained from the lyophilized cells of the obtained MmEV4 gene expression strain, AsEV4b gene expression strain, and pYES NT/A-transduced strain, as follows. Microbial oils A to C obtained were converted into fatty acid methyl esters and subjected to fatty acid analysis.

According to the method of Folch et al. (J. Biological and Chemistry. 226: 497-509 (1957)), total lipids were extracted from the lyophilized cells with chloroform: methanol (2:1, v/v). The total lipids thus obtained were methyl-esterified to obtain a fatty acid methyl ester (FAME). The FAME thus obtained was subjected to FAME analysis by gas chromatography. The conditions for the gas chromatography were set as follows.
- Column: DB-17ht 0.250 mm × 30 m, film thickness 0.15 µm (Agilent Technologies Japan, Ltd.)
- Carrier gas conditions: helium 1.0 ml/min, separation ratio 50:1
- Column temperature conditions: 5 minutes at 180°C, temperature rise up to 320°C at 1°C/min, 10 minutes at 320°C
- Detection: FID
- Detector temperature: 320°C
- Inlet temperature: 300°C
- Injection volume: 1 µL

The conditions for gas chromatography-mass spectrometry were set as follows.
Gas Chromatography Separation Conditions
   - Apparatus: Agilent 7890B (Agilent Technologies Japan, Ltd.)
   - Column: DB-17ht 0.250 mm × 30 m, film thickness 0.15 µm (Agilent Technologies Japan, Ltd.)
   - Carrier gas conditions: constant flow, helium 1.0 ml/min, separation ratio 10:1
   - Column temperature conditions: 5 minutes at 180°C, temperature rise up to 300°C at 4°C/min, 10 minutes at 300°C
   - Inlet temperature: 300°C
   - Injection volume: 1 µL
Mass Spectrometry Conditions
   - Detection: APCI source ((Atmospheric Pressure Gas Chromatography: APGC) (Waters Corporation)
   - Mass Spectrometer: A Quadrupole, A Collision Cell, Time of Flight Analyzer (Xevo G2-XS QTof) (Waters Corporation)
   - Ion source: API, Positive mode, Sensitive mode
   - Data acquisition mode: MS^{E}
   - Ionization process: wet (ethanol)
   - Collision energy: low energy 6 eV, high energy 20 to 50 eV m/z 50-650
   - Corona voltage: 3.0 µA
   - Source temperature: 150°C
   - Cone voltage: 10 V
   - Gas flow rate: 100 L/hr
   - Auxiliary gas flow rate: 100 L/hr
   - Analysis software: MassLynx

Information about individual compounds are shown below.

**[Table 4]**

| Table 4: Compound information | | | |
|---|---|---|---|
| Retention time (min) | Presumed chemical formula | Presumed compound | Measured value (*m*/*z*) [M+H]⁺ |
| 3.52 | C₁₉H₃₈O₂ | C18:0ME | 299.2973 |
| 3.583 | C₁₉H₃₆O₂ | C18:1ME | 297.2805 |
| 4.264 | C₂₀H₄₀O₂ | C19:0ME | 313.3083 |
| 6.266 | C₂₁H₃₂O₂ | C20:5ME | 317.2494 |
| 8.554 | C₂₃H₄₆O₂ | C22:0ME | 355.3546 |
| 8.691 | C₂₃H₄₄O₂ | C22:1ME | 353.3386 |
| 10.304 | C₂₃H₃₆O₂ | C22:5ME | 345.2795 |
| 17.58 | C₂₅H₄₀O₂ | C24:5ME | 373.3072 |
| 18.034 | C₂₃H₃₆O₃ | | 361.271 |
| 25.473 | C₂₇H₅₄O₂ | C26:0ME | 411.4163 |
| 30.678 | C₂₇H₄₄O₂ | C26:5ME | 401.3388 |
| 45.253 | C₂₉H₅₈O₂ | C28:0ME | 439.4487 |
| 68.464 | C₃₁H₆₂O₂ | C30:0ME | 467.4808 |
| 71.278 | C₃₁H₅₂O₂ | C30:5ME | 457.4039 |
| 76.775 | C₃₃H₅₆O₂ | C32:5ME | 485.4377 |

| | | | |
|---|---|---|---|
| (ME in the presumed compound represents a methyl ester) | | | |

A compound corresponding to an APGC retention time of 18.034 minutes was not able to be presumed, so this compound was designated as impurity X. Information about an impurity is given below.
- APGC retention time: 18.034 minutes
- Presumed chemical formula: C₂₃H₃₆O₃
- Measured m/z: [M+H]⁺ 361.2710

Fatty acid compositions of microbial oils A to C are shown in Table 5.

In microbial oil A obtained from the MmELOVL4 expression strain, C30:5, C32:5, C34:5 and C36:5, which are EPA-derived VLC-PUFAs, were not able to be detected.

On the other hand, C32:5 and C34:5, which are chain elongated fatty acids of EPA, were able to be detected in microbial oil B obtained from the AsELOVL4b expression strain. Microbial oil B comprised 0.32 area% and 0.46 area% of C32:5 and C34:5, respectively, to the total fatty acids. A ratio of stearic acid, which is a fatty acid derived from the host budding yeast, to the total EPA-derived VLC-PUFAs (sum of C32:5 and C34:5) ((stearic acid)/(VLC-PUFA)) was 7.20. Further, a ratio of impurity X to the total EPA-derived VLC-PUFAs (sum of C32:5 and C34:5) ((impurity X)/(VLC-PUFA)) was 0.26. In addition, in microbial oil C obtained from the pYES2 NT/A-transduced strain, C30:5, C32:5, C34:5, and C36:5, which are EPA-derived VLC-PUFAs were not able to be detected.

**[Table 5]**

| Table 5: Fatty acid composition | | | |
|---|---|---|---|
| | Mm4_2 | AsEV4b_2 | pYES2 NT/A_1 |
| | Comparative Example 1 | Comparative Example 2 | Comparative Example 3 |
| | Oil A | Oil B | Oil C |
| C15:0 | 0.44 | 0.49 | 0.50 |
| C15:1 | 0.14 | 0.26 | 0.00 |
| C16:0 | 14.53 | 10.56 | 12.78 |
| C16:1 | 17.44 | 17.44 | 18.42 |
| C18:0 | 7.44 | 5.61 | 7.64 |
| C18:1 | 17.30 | 19.45 | 19.12 |
| C19:0 | 0.15 | 0.00 | 0.00 |
| C20:5 | 29.68 | 30.88 | 30.27 |
| C22:0 | 0.00 | 0.00 | 0.00 |
| C22:1 | 0.00 | 0.00 | 0.00 |
| C22:5 | 0.54 | 1.08 | 0.00 |
| C24:5 | 0.00 | 0.35 | 0.00 |
| C23H36O3 | 0.09 | 0.20 | 0.00 |
| C26:0 | 0.00 | 0.65 | 0.00 |
| C26:5 | 0.00 | 0.12 | 0.00 |
| C28:0 | 0.00 | 0.13 | 0.00 |
| C28:5 | 0.00 | 0.00 | 0.00 |
| C30:0 | 0.00 | 0.00 | 0.00 |
| C30:5 | 0.00 | 0.00 | 0.00 |
| C32:5 | 0.00 | 0.32 | 0.00 |
| C34:5 | 0.00 | 0.46 | 0.00 |
| C36:5 | 0.00 | 0.00 | 0.00 |
| others | 12.26 | 12.00 | 11.27 |
| C18:0/C30:5 | - | - | - |
| C18:0/C32:5 | - | 17.34 | - |
| C18:0/C34:5 | - | 12.31 | - |
| C18:0/C36:5 | - | - | - |
| C18:0/VLC | - | 7.20 | - |
| | | | |
| (Impurity X)/C30:5 | - | - | - |
| (Impurity X)/C32:5 | - | 0.63 | - |
| (Impurity X)/C34:5 | - | 0.44 | - |
| (Impurity X)/C36:5 | - | - | - |
| (Impurity X)/VLC | - | 0.26 | - |

### Example 5: Production of VLC-PUFAs by Constitutively Expressing AsELOVL4b Gene Expression Strain

From the constitutive AsELOVL4b gene expression strain prepared by the method described in Example 3, microbial oil D was obtained by the method described in Example 4 and subjected to fatty acid analysis. For comparison, microbial oil B obtained in Example 4 (inducible promoter (Gal1 promoter) AsELOVL4b gene expression strain) was used.

The fatty acid compositions of microbial oils B and D are shown in Table 6.

In microbial oil D obtained from the constitutively expressing AsELOVL4b expression strain, C32:5 and C34:5, which are EPA-derived VLC-PUFAs were able to be detected. Microbial oil D comprised 0.32 area% and 0.28 area% of C32:5 and C34:5, respectively, to the total area value of all fatty acids. A ratio of stearic acid, which is a fatty acid derived from the host budding yeast, to the total EPA-derived VLC-PUFAs (sum of C32:5 and C34:5) ((stearic acid)/(VLC-PUFA)) was 15.5. Further, a ratio of impurity X to the total EPA-derived VLC-PUFAs (sum of C32:5 and C34:5) ((impurity X)/(VLC-PUFA)) was 0. 17.

The above results show that the use of the constitutive promoter did not increase the ratio of VLC-PUFAs comprised in the obtainable microbial oil, but increased the ratio of host-derived fatty acids.

**[Table 6]**

| Table 6: Fatty acid composition (constitutive promoter and inducible promoter) | | |
|---|---|---|
| | PGK1_1 | Gal1_2 |
| | Comparative Example 4 | Comparative Example 2 |
| | Oil D | Oil B |
| C15:0 | 0.72 | 0.49 |
| C15:1 | 0.47 | 0.26 |
| C16:0 | 12.09 | 10.56 |
| C16:1 | 15.40 | 17.44 |
| C18:0 | 9.36 | 5.61 |
| C18:1 | 13.47 | 19.45 |
| C19:0 | 0.74 | 0.00 |
| C20:5 | 31.21 | 30.88 |
| C22:0 | 0.24 | 0.00 |
| C22:1 | 0.21 | 0.00 |
| C22:5 | 1.28 | 1.08 |
| C24:5 | 0.03 | 0.35 |
| C23H36O3 | 0.10 | 0.20 |
| C26:0 | 1.85 | 0.65 |
| C26:5 | 0.00 | 0.12 |
| C28:0 | 0.20 | 0.13 |
| C28:5 | 0.00 | 0.00 |
| C30:0 | 0.00 | 0.00 |
| C30:5 | 0.00 | 0.00 |
| C32:5 | 0.32 | 0.32 |
| C34:5 | 0.28 | 0.46 |
| C36:5 | 0.00 | 0.00 |
| others | 12.11 | 12.00 |
| | | |
| C18:0/C30:5 | - | - |
| C18:0/C32:5 | 29.24 | 17.34 |
| C18:0/C34:5 | 32.87 | 12.31 |
| C18:0/C36:5 | - | - |
| C18:0/VLC | 15.47 | 7.20 |
| | | |
| (Impurity X)/C30:5 | | |
| (Impurity X)/C32:5 | 0.31 | 0.63 |
| (Impurity X)/C34:5 | 0.35 | 0.44 |
| (Impurity X)/C36:5 | | |
| (Impurity X)/VLC | 0.17 | 0.26 |

### Example 6: Examination of Expression Induction Timing and Production of VLC-PUFAs by AsELOVL4b Gene Expression Strain

The AsELOVL4b gene expression strain prepared by the method described in Example 2 was cultured in 5 ml of SD-ura liquid medium at 28°C and 180rpm for 16 hours. 0.1 ml of culture solutions were inoculated into 5 ml of uracil-restricted raffinose media (Minimal SD Base/Raf (manufactured by Takara Bio, Inc.) 37 g/L, -Ura DO Supplement 0.77 g/L, adjusted to pH 5.8) and cultured until the turbidity values of the culture solutions reached 1.0, 2.3, 4.8, 9.6, and 11.2, respectively. Thereafter, a 40% galactose solution was added in an amount of 1/20 times the volume of the culture solution, and EPA was further added in an amount of 1/1000 times the volume of the culture medium (5 µl). After addition of the galactose solution and EPA, cells were further cultured for 2 to 5 days, and the entire culture solution was aseptically collected in a 15 ml centrifuge tube and centrifuged at 3000 xg for 3 minutes to collect the cells. Thereafter, the cells were frozen at - 80°C and then subjected to a lyophilizer to obtain lyophilized cells. According to the method described in Example 4, microbial oils E to I were obtained from the lyophilized cells of the AsELOVL4b expression strains with the turbidity values of 1.0, 2.3, 4.8, 9.6, and 11.2 in the culture solutions in the uracil-restricted raffinose media, respectively, and subjected to fatty acid analysis. The fatty acid compositions of microbial oils E to I are shown in Table 7.

C30:5, C32:5, and C34:5, which are EPA-derived VLC-PUFAs, were able to be detected in microbial oils E to H. C32:5 and C34:5, which are EPA-derived VLC-PUFAs, were able to be detected in microbial oil I.

In microbial oils F, G, and H, ratios of C30:5, C32:5, and C34:5 to the total area value of total fatty acids were higher than in microbial oils E and I.

Further, ratios of stearic acid, which is a fatty acid derived from the host budding yeast, to the total EPA-derived VLC-PUFAs (sum of C30:5, C32:5, and C34:5) ((stearic acid)/(VLC-PUFA)) were also low compared to microbial oils E and I, and were able to be reduced to less than 6.

Furthermore, ratios of impurity X to the total EPA-derived VLC-PUFAs (sum of C32:5 and C34:5) ((impurity X)/(VLC-PUFA)) were less than 0.16.

The above results show that using an inducible promoter and inducing the expression when the turbidity value of the culture solution was from 2.3 to 9.6 increased the ratio of VLC-PUFAs comprised in the obtainable microbial oil, reduced the ratio of host-derived fatty acids, and reduced the ratio of impurity X.

**[Table 7]**

| Table 7: Fatty acid compositions (fatty acid compositions of microbial oils with different expression induction times: 5 types) | | | | | |
|---|---|---|---|---|---|
| | Turbidity 1.0 | Turbidity 2.3 | Turbidity 4.8 | Turbidity 9.6 | Turbidity 11.2 |
| | Comparative Example 2 | Example 1 | Example 2 | Example 3 | Comparative Example 6 |
| | Oil B | Oil F | Oil G | Oil H | Oil I |
| C15:0 | 0.49 | 0.40 | 0.25 | 0.31 | 0.26 |
| C15:1 | 0.26 | 0.23 | 0.10 | 0.17 | 0.18 |
| C16:0 | 10.56 | 10.86 | 12.60 | 14.03 | 8.52 |
| C16:1 | 17.44 | 16.86 | 14.75 | 16.33 | 16.89 |
| C18:0 | 5.61 | 5.08 | 7.57 | 5.28 | 3.55 |
| C18:1 | 19.45 | 15.28 | 13.97 | 12.96 | 14.36 |
| C19:0 | 0.00 | 0.19 | 0.12 | 0.32 | 0.18 |
| C20:5 | 30.88 | 29.24 | 28.03 | 29.56 | 28.65 |
| C22:0 | 0.00 | 0.00 | 0.08 | 0.00 | 0.00 |
| C22:1 | 0.00 | 0.00 | 0.00 | 0.00 | 0.00 |
| C22:5 | 1.08 | 3.07 | 3.27 | 2.68 | 0.41 |
| C24:5 | 0.35 | 0.70 | 1.09 | 0.63 | 0.08 |
| C23H36O3 | 0.20 | 0.40 | 0.24 | 0.44 | 0.07 |
| C26:0 | 0.65 | 0.62 | 1.02 | 0.56 | 0.59 |
| C26:5 | 0.12 | 0.06 | 0.13 | 0.19 | 0.00 |
| C28:0 | 0.13 | 0.15 | 0.38 | 0.10 | 0.00 |
| C28:5 | 0.00 | 0.00 | 0.00 | 0.00 | 0.00 |
| C30:0 | 0.00 | 0.00 | 0.07 | 0.00 | 0.00 |
| C30:5 | 0.00 | 0.34 | 0.28 | 0.31 | 0.00 |
| C32:5 | 0.32 | 1.60 | 2.18 | 1.14 | 0.08 |
| C34:5 | 0.46 | 2.54 | 2.73 | 1.37 | 0.06 |
| C36:5 | 0.00 | 0.00 | 0.00 | 0.00 | 0.00 |
| others | 12.00 | 12.38 | 11.14 | 13.63 | 13.54 |
| | | | | | |
| C18:0/C30:5 | - | 15.00 | 27.08 | 17.13 | - |
| C18:0/C32:5 | 17.34 | 3.17 | 3.47 | 4.62 | 41.92 |
| C18:0/C34:5 | 12.31 | 2.00 | 2.77 | 3.84 | 54.99 |
| C18:0/C36:5 | - | - | - | - | - |
| C18:0/VLC | 7.20 | 1.13 | 1.46 | 1.87 | 23.79 |
| | | | | | |
| (Impurity X)/C30:5 | - | 1.18 | 0.87 | 1.44 | - |
| (Impurity X)/C32:5 | 0.63 | 0.25 | 0.11 | 0.39 | 0.79 |
| (Impurity X)/C34:5 | 0.44 | 0.16 | 0.09 | 0.32 | 1.04 |
| (Impurity X)/C36:5 | | | | | |
| (Impurity X)/VLC | 0.26 | 0.09 | 0.05 | 0.16 | 0.45 |

### Example 7: Examination of PUFAs to be Added

Using the method described in Example 6, lyophilized cells of a CgELOVL4-expressing strain with a turbidity of 4.8 in culture solution in uracil-restricted raffinose medium were obtained by culturing the AsELOVL4b gene expression strain prepared by the method described in Example 2, with the added PUFAs changed to arachidonic acid (ARA). Microbial oil J was then obtained from the lyophilized cells and subjected to fatty acid analysis.

The fatty acid composition of microbial oil J is shown in Table 8.

In microbial oil J obtained from the AsELOVL4b expression strain, C30:4, C32:4, and C34:4, which are ARA-derived VLC-PUFAs, were able to be detected. Microbial oil J comprised 0.58 area%, 0.86 area%, and 0.15 area% of C30:4, C32:4, and C34:4, respectively, to the total area value of all fatty acids.

A ratio of stearic acid, which is a fatty acid derived from the host budding yeast, to the total ARA-derived VLC-PUFAs (sum of C30:4, C32:4, and C34:4) ((stearic acid)/(VLC-PUFA)) was 2.4. Furthermore, a ratio of impurity X2 to the total ARA-derived VLC-PUFA (sum of C32:5 and C34:5) ((impurity X2)/(VLC-PUFA)) was less than 0.16.

Thus, it was confirmed that VLC-PUFAs can be produced using PUFAs other than EPA as substrates.
[Table 8]

**Table 8: Fatty acid composition (VLC-PUFA production starting from ARA)**

| | |
|---|---|
| | ARA |
| | **Example 4** |
| | Oil J |
| C15:0 | 0.55 |
| C15:1 | 0.08 |
| C16:0 | 7.29 |
| C16:1 | 26.56 |
| C18:0 | 3.75 |
| C18:1 | 14.58 |
| C19:0 | 0.00 |
| C20:4 | 30.23 |
| C22:0 | 0.00 |
| C22:1 | 0.00 |
| C22:4 | 1.33 |
| C24:4 | 0.25 |
| C23H36O3 | 0.24 |
| C26:0 | 0.46 |
| C26:4 | 0.14 |
| C28:0 | 0.21 |
| C28:4 | 0.00 |
| C30:0 | 0.00 |
| C30:4 | 0.58 |
| C32:4 | 0.86 |
| C34:4 | 0.15 |
| C36:4 | 0.00 |
| others | 12.75 |
| | |
| C18:0/C30:4 | 6.45 |
| C18:0/C32:4 | 4.37 |
| C18:0/C34:4 | 24.20 |
| C18:0/C36:6 | - |
| C18:0/VLC | 2.35 |
| | |
| (Impurity Y)/C30:4 | 0.41 |
| (Impurity Y)/C32:4 | 0.28 |
| (Impurity Y)/C34:4 | 1.55 |
| (Impurity Y)/C36:4 | - |
| (Impurity Y)/VLC | 0.15 |

### Example 8: Production of VLC-PUFAs by CgELOVL4a Gene Expression Strain

An African sharptooth catfish (*Clarias gariepinus*)-derived ELOVL4a gene (ACCESSION No. KY801284) (hereinafter referred to as CgELOVL4a) with a codon optimized for expression in budding yeast was artificially synthesized. A synthesized DNA fragment was used as a template to amplify a CgELOVL4a gene fragment using a primer pair of SEQ ID NO: 9 and SEQ ID NO: 10 shown in Table 3. The amplified fragments were fused with a pYES2 NT/A vector (Thermo Fisher Scientific Inc.) cleaved with restriction enzymes HindIII and KpnI using the In-Fusion HD Cloning Kit (Takara Bio, Inc.) to construct a plasmid for CgELOV4 gene expression.

This plasmid for expression comprises an insert sequence wherein a Gal1 promoter sequence, the CgELOVL4a gene, and a CYC1 terminator sequence are linked in this order, and a pYES2 NT/A vector sequence comprising a ura3 gene.

The prepared plasmid for CgELOVL4a expression was transduced into a budding yeast *S. cerevisiae* INVSc1 strain (Thermo Fisher Scientific Inc.) by the lithium acetate method described in Example 1, and a gene recombinant was obtained using uracil requirement as an indicator.

From the lyophilized cells of the CgELOVL4a gene expression strain obtained by culturing the obtained CgELOVL4a gene expression strain until the turbidity value of the culture solution in the uracil-restricted raffinose medium reached 4.8, by the method described in Example 6, microbial oil K was obtained and subjected to fatty acid analysis.

The fatty acid composition of microbial oil K is shown in Table 9.

In microbial oil K obtained from the CgELOVL4a expression strain, C32:5, C34:5, and C36:5, which are EPA-derived VLC-PUFAs, were able to be detected. Microbial oil K comprised 0.16 area%, 0.79 area%, and 0.59 area% of C32:5, C34:5, and C36:5, respectively, to the total area value of all fatty acids. A ratio of stearic acid, which is a fatty acid derived from the host budding yeast to the total EPA-derived VLC-PUFAs (sum of C32:5, C34:5, and C36:5) ((stearic acid)/(VLC-PUFA)) was 4.8. Further, a ratio of impurity X to the total EPA-derived VLC-PUFAs (sum of C32:5, C34:5, and C36:5) ((impurity X)/(VLC-PUFA)) was 0.12.

Thus, it was confirmed that by using ELOVL4 not derived from black seabream, a new VLC-PUFA-comprising microbial oil comprising C36:5 can be obtained while maintaining the effects of the invention.
[Table 9]

**Table 9: Fatty acid composition (VLC-PUFA production in CgEV4 gene expression strain)**

| | |
|---|---|
| | CgEV4 |
| | **Example 5** |
| | OilK |
| C15:0 | 0.43 |
| C15:1 | 0.20 |
| C16:0 | 12.66 |
| C16:1 | 14.81 |
| C18:0 | 7.30 |
| C18:1 | 9.57 |
| C19:0 | 0.16 |
| C20:5 | 28.70 |
| C22:0 | 0.09 |
| C22:1 | 0.00 |
| C22:5 | 4.09 |
| C24:5 | 1.26 |
| C23H36O3 | 0.19 |
| C26:0 | 1.57 |
| C26:5 | 0.34 |
| C28:0 | 0.45 |
| C28:5 | 0.00 |
| C30:0 | 0.14 |
| C30:5 | 0.00 |
| C32:5 | 0.16 |
| C34:5 | 0.79 |
| C36:5 | 0.59 |
| others | 16.44 |
| | |
| C18:0/C30:5 | - |
| C18:0/C32:5 | 46.51 |
| C18:0/C34:5 | 9.29 |
| C18:0/C36:5 | 16.27 |
| C18:0/VLC | 4.77 |
| | |
| (Impurity X)/C30:5 | |
| (Impurity X)/C32:5 | 1.21 |
| (Impurity X)/C34:5 | 0.24 |
| (Impurity X)/C36:5 | 0.32 |
| (Impurity X)/VLC | 0.12 |

## Claims

1. A composition comprising:
a cell expressing a PUFA chain elongase and/or a crushed material thereof; and
a polyunsaturated fatty acid having at least 30 carbons and/or a derivative thereof.

2. The composition according to claim 1, wherein
the polyunsaturated fatty acid having at least 30 carbons comprises at least one polyunsaturated fatty acid selected from the group consisting of the following:
(1) a polyunsaturated fatty acid having 30 carbons;
(2) a polyunsaturated fatty acid having 32 carbons;
(3) a polyunsaturated fatty acid having 34 carbons; and
(4) a polyunsaturated fatty acid having 36 carbons.

3. The composition according to claim 2, wherein a content of one polyunsaturated fatty acid selected from the group consisting of (1) to (4) and/or a derivative thereof is at least 0.5 area%.

4. The composition according to claim 2 or 3, wherein a content of the polyunsaturated fatty acids of (1) to (4) and/or derivatives thereof is at least 1.0 area%, at least 1.5 area%, at least 2.0 area%, or at least 2.5 area%.

5. The composition according to any one of claims 2 to 4, wherein a ratio of a content of stearic acid and/or a derivative thereof to a total content of the polyunsaturated fatty acids of (1) to (4) and/or the derivatives thereof is less than 6.0, less than 5.0, less than 2.5, or less than 2.0.

6. The composition according to any one of claims 2 to 5, wherein the polyunsaturated fatty acid and/or the derivative thereof in the composition satisfies at least one of the following conditions:
(1) a ratio of a content of stearic acid and/or a derivative thereof to a content of the polyunsaturated fatty acid having 30 carbons and/or the derivative thereof is less than 50, less than 30, less than 20, or less than 10;
(2) a ratio of a content of stearic acid and/or a derivative thereof to a content of the polyunsaturated fatty acid having 32 carbons and/or the derivative thereof is less than 15, less than 10, or less than 5;
(3) a ratio of a content of stearic acid and/or a derivative thereof to a content of the polyunsaturated fatty acid having 34 carbons and/or the derivative thereof is less than 10, less than 5, or less than 2; and
(4) a ratio of a content of stearic acid and/or a derivative thereof to a content of the polyunsaturated fatty acid having 36 carbons and/or the derivative thereof is less than 20.

7. The composition according to any one of claims 1 to 6, comprising an impurity having a chemical formula of C23H36O3.

8. The composition according to any one of claims 1 to 7, wherein a content of a cholesterol ester, phospholipid, glycolipid, monoglyceride, diglyceride, and triglyceride is at least 70 wt.% of a total weight of the composition.

9. The composition according to any one of claims 2 to 8, wherein at least one fatty acid of a constituent fatty acid of a cholesterol ester, phospholipid, glycolipid, monoglyceride, diglyceride, and triglyceride is at least one polyunsaturated fatty acid of (1) to (4).

10. The composition according to any one of claims 2 to 9, wherein at least one fatty acid of constituent fatty acids of a cholesterol ester, phospholipid, glycolipid, monoglyceride, diglyceride, and triglyceride is at least one polyunsaturated fatty acid of (1) to (4), and of the constituent fatty acids of a cholesterol ester, phospholipid, glycolipid, monoglyceride, diglyceride, and triglyceride, a fatty acid other than the polyunsaturated fatty acids of (1) to (4) is a fatty acid having from 8 to 22 hydrocarbons.

11. The composition according to any one of claims 1 to 10, comprising a PUFA having at least 0.5 area%.

12. The composition according to claim 11, wherein the PUFA comprises a non-productive PUFA externally added.

13. The composition according to any one of claims 1 to 12, wherein the cell is yeast.

14. The composition according to any one of claims 1 to 13, wherein
the PUFA chain elongase is one of the following:
a) non-mammalian ELOVL4; and
b) a protein comprising an amino acid sequence having at least 90% sequence identity to an amino acid sequence encoding non-mammalian ELOVL4 and having PUFA chain elongation activity.

15. The composition according to any one of claims 1 to 14, wherein a gene of the PUFA chain elongase is expressed by an inducible promoter.

16. Crude oil comprising a polyunsaturated fatty acid having at least 30 carbons and/or a derivative thereof at a content of at least 1.0 area%, at least 1.5 area%, at least 2.0 area%, or at least 2.5 area%.

17. The crude oil according to claim 16, wherein
the polyunsaturated fatty acid having at least 30 carbons comprises at least one polyunsaturated fatty acid selected from the group consisting of the following:
(1) a polyunsaturated fatty acid having 30 carbons;
(2) a polyunsaturated fatty acid having 32 carbons;
(3) a polyunsaturated fatty acid having 34 carbons; and
(4) a polyunsaturated fatty acid having 36 carbons.

18. The crude oil according to claim 17, wherein a content of one polyunsaturated fatty acid selected from the group consisting of (1) to (4) and/or a derivative thereof is at least 0.5 area%.

19. The crude oil according to claim 17 or 18, wherein a content of the polyunsaturated fatty acids of (1) to (4) and/or derivatives thereof is at least 1.0 area%, at least 1.5 area%, at least 2.0 area%, or at least 2.5 area%.

20. The crude oil according to any one of claims 17 to 19, wherein a ratio of a content of stearic acid and/or a derivative thereof to a total content of the polyunsaturated fatty acids of (1) to (4) and/or the derivatives thereof is less than 6.0, less than 5.0, less than 2.5, or less than 2.0.

21. The crude oil according to any one of claims 16 to 20, wherein
the polyunsaturated fatty acid and/or the derivative thereof in the crude oil satisfies at least one of the following conditions:
(1) a ratio of a content of stearic acid and/or a derivative thereof to a content of the polyunsaturated fatty acid having 30 carbons and/or the derivative thereof is less than 50, less than 30, less than 20, or less than 10;
(2) a ratio of a content of stearic acid and/or a derivative thereof to a content of the polyunsaturated fatty acid having 32 carbons and/or the derivative thereof is less than 15, less than 10, or less than 5;
(3) a ratio of a content of stearic acid and/or a derivative thereof to a content of the polyunsaturated fatty acid having 34 carbons and/or the derivative thereof is less than 10, less than 5, or less than 2; and
(4) a ratio of a content of stearic acid and/or a derivative thereof to a content of the polyunsaturated fatty acid having 36 carbons and/or the derivative thereof is less than 20.

22. The crude oil according to any one of claims 16 to 21, comprising an impurity having a chemical formula of C23H36O3.

23. The crude oil according to any one of claims 16 to 22, wherein a content of a cholesterol ester, phospholipid, glycolipid, monoglyceride, diglyceride, and triglyceride is at least 70 wt.% of a total weight.

24. The crude oil according to any one of claims 17 to 23, wherein at least one fatty acid of a constituent fatty acid of a cholesterol ester, phospholipid, glycolipid, monoglyceride, diglyceride, and triglyceride is at least one polyunsaturated fatty acid of (1) to (4).

25. The crude oil according to any one of claims 17 to 24, wherein at least one fatty acid of constituent fatty acids of a cholesterol ester, phospholipid, glycolipid, monoglyceride, diglyceride, and triglyceride is at least one polyunsaturated fatty acid of (1) to (4), and of the constituent fatty acids of a cholesterol ester, phospholipid, glycolipid, monoglyceride, diglyceride, and triglyceride, a fatty acid other than the polyunsaturated fatty acids of (1) to (4) is a fatty acid having from 8 to 22 hydrocarbons.

26. The crude oil according to any one of claims 16 to 25, comprising a PUFA having at least 0.5 area%.

27. The crude oil according to claim 26, wherein the PUFA comprises a non-productive PUFA externally added.

28. The crude oil according to any one of claims 16 to 27, wherein the crude oil is a crude oil derived from yeast.

29. The crude oil according to claim 28, wherein the yeast is a yeast expressing a PUFA chain elongase gene.

30. The crude oil according to claim 29, wherein the PUFA chain elongase gene is expressed by an inducible promoter.

31. The crude oil according to claim 29 or 30, wherein
the PUFA chain elongase gene comprises:
a) a polynucleotide encoding non-mammalian ELOVL4; or
b) a polynucleotide having a nucleotide sequence having at least 90% sequence identity to a nucleotide sequence of the polynucleotide encoding non-mammalian ELOVL4 and encoding a protein having PUFA chain elongation activity.

32. A composition comprising a polyunsaturated fatty acid having at least 30 carbons and/or a derivative thereof, wherein
a content of the polyunsaturated fatty acid and/or the derivative thereof is at least 1.0 area%, at least 1.5 area%, at least 2.0 area%, or at least 2.5 area%, and
a total content of an acylcholesterol, phospholipid, glycolipid, monoglyceride, diglyceride, and triglyceride is at least 70 wt.%.

33. The composition according to claim 32, wherein
the polyunsaturated fatty acid having at least 30 carbons comprises at least one polyunsaturated fatty acid selected from the group consisting of the following:
(1) a polyunsaturated fatty acid having 30 carbons;
(2) a polyunsaturated fatty acid having 32 carbons;
(3) a polyunsaturated fatty acid having 34 carbons; and
(4) a polyunsaturated fatty acid having 36 carbons.

34. The composition according to claim 33, wherein a content of one polyunsaturated fatty acid selected from the group consisting of (1) to (4) and/or a derivative thereof is at least 0.5 area%.

35. The composition according to claim 33 or 34, comprising the polyunsaturated fatty acid having at least 30 carbons and/or the derivative thereof, wherein
the polyunsaturated fatty acid and/or the derivative thereof comprises the polyunsaturated fatty acids of (1) to (4) and/or derivatives thereof at a content of at least 1.0 area%, at least 1.5 area%, at least 2.0 area%, or at least 2.5 area%.

36. The composition according to any one of claims 33 to 35, wherein a ratio of a content of stearic acid and/or a derivative thereof to a total content of the polyunsaturated fatty acids of (1) to (4) and/or the derivatives thereof is less than 6.0, less than 5.0, less than 2.5, or less than 2.0.

37. The composition according to any one of claims 33 to 36, wherein
the polyunsaturated fatty acid and/or the derivative thereof in the composition satisfies at least one of the following conditions:
(1) a ratio of a content of stearic acid and/or a derivative thereof to a content of the polyunsaturated fatty acid having 30 carbons and/or the derivative thereof is less than 50, less than 30, less than 20, or less than 10;
(2) a ratio of a content of stearic acid and/or a derivative thereof to a content of the polyunsaturated fatty acid having 32 carbons and/or the derivative thereof is less than 15, less than 10, or less than 5;
(3) a ratio of a content of stearic acid and/or a derivative thereof to a content of the polyunsaturated fatty acid having 34 carbons and/or the derivative thereof is less than 10, less than 5, or less than 2; and
(4) a ratio of a content of stearic acid and/or a derivative thereof to a content of the polyunsaturated fatty acid having 36 carbons and/or the derivative thereof is less than 20.

38. The composition according to any one of claims 33 to 37, comprising an impurity of a chemical formula of C23H36O3.

39. The composition according to any one of claims 32 to 38, wherein a content of a cholesterol ester, phospholipid, glycolipid, monoglyceride, diglyceride, and triglyceride is at least 70 wt.% of a total weight of the composition.

40. The composition according to any one of claims 33 to 39, wherein at least one fatty acid of a constituent fatty acid of a cholesterol ester, phospholipid, glycolipid, monoglyceride, diglyceride, and triglyceride is at least one polyunsaturated fatty acid of (1) to (4).

41. The composition according to any one of claims 33 to 40, wherein at least one fatty acid of constituent fatty acids of a cholesterol ester, phospholipid, glycolipid, monoglyceride, diglyceride, and triglyceride is at least one polyunsaturated fatty acid of (1) to (4), and of the constituent fatty acids of a cholesterol ester, phospholipid, glycolipid, monoglyceride, diglyceride, and triglyceride, a fatty acid other than the polyunsaturated fatty acids of (1) to (4) is a fatty acid having from 8 to 22 hydrocarbons.

42. The composition according to any one of claims 32 to 41, comprising a PUFA having at least 0.5 area%.

43. The composition according to claim 42, wherein the PUFA comprises a non-productive PUFA externally added.

44. Microbial oil comprising a polyunsaturated fatty acid having at least 30 carbons and/or a derivative thereof at a content of at least 1.0 area%, at least 1.5 area%, at least 2.0 area%, or at least 2.5 area%.

45. The microbial oil according to claim 44, wherein
the polyunsaturated fatty acid having at least 30 carbons comprises at least one polyunsaturated fatty acid selected from the group consisting of the following:
(1) a polyunsaturated fatty acid having 30 carbons;
(2) a polyunsaturated fatty acid having 32 carbons;
(3) a polyunsaturated fatty acid having 34 carbons; and
(4) a polyunsaturated fatty acid having 36 carbons.

46. The microbial oil according to claim 45, wherein a content of one polyunsaturated fatty acid selected from the group consisting of (1) to (4) and/or a derivative thereof is at least 0.5 area%.

47. The microbial oil according to claim 45 or 46, comprising the polyunsaturated fatty acid having at least 30 carbons and/or the derivative thereof, wherein
the polyunsaturated fatty acid and/or the derivative thereof comprises the polyunsaturated fatty acids of (1) to (4) and/or derivatives thereof at a content of at least 1.0 area%, at least 1.5 area%, at least 2.0 area%, or at least 2.5 area%.

48. The microbial oil according to any one of claims 45 to 47, wherein a ratio of a content of stearic acid and/or a derivative thereof to a total content of the polyunsaturated fatty acids of (1) to (4) and/or the derivatives thereof in the microbial oil is less than 6.0, less than 5.0, less than 2.5, or less than 2.0.

49. The microbial oil according to any one of claims 45 to 48, wherein
the polyunsaturated fatty acid and/or the derivative thereof in the microbial oil satisfies at least one of the following conditions:
(1) a ratio of a content of stearic acid and/or a derivative thereof to a content of the polyunsaturated fatty acid having 30 carbons and/or the derivative thereof is less than 50, less than 30, less than 20, or less than 10;
(2) a ratio of a content of stearic acid and/or a derivative thereof to a content of the polyunsaturated fatty acid having 32 carbons and/or the derivative thereof is less than 15, less than 10, or less than 5;
(3) a ratio of a content of stearic acid and/or a derivative thereof to a content of the polyunsaturated fatty acid having 34 carbons and/or the derivative thereof is less than 10, less than 5, or less than 2; and
(4) a ratio of a content of stearic acid and/or a derivative thereof to a content of the polyunsaturated fatty acid having 36 carbons and/or the derivative thereof is less than 20.

50. The microbial oil according to any one of claims 44 to 48, comprising an impurity having a chemical formula of C23H36O3.

51. The microbial oil according to any one of claims 44 to 50, wherein a content of a cholesterol ester, phospholipid, glycolipid, monoglyceride, diglyceride, and triglyceride is at least 70 wt.% of a total weight.

52. The microbial oil according to any one of claims 44 to 51, wherein a content of a cholesterol ester, phospholipid, glycolipid, monoglyceride, diglyceride, and triglyceride is at least 70 wt.% of a total weight.

53. The microbial oil according to any one of claims 45 to 52, wherein at least one fatty acid of a constituent fatty acid of a cholesterol ester, phospholipid, glycolipid, monoglyceride, diglyceride, and triglyceride is at least one polyunsaturated fatty acid of (1) to (4).

54. The microbial oil according to any one of claims 45 to 53, wherein at least one fatty acid of constituent fatty acids of a cholesterol ester, phospholipid, glycolipid, monoglyceride, diglyceride, and triglyceride is at least one polyunsaturated fatty acid of (1) to (4), and of the constituent fatty acids of a cholesterol ester, phospholipid, glycolipid, monoglyceride, diglyceride, and triglyceride, a fatty acid other than the polyunsaturated fatty acids of (1) to (4) is a fatty acid having from 8 to 22 hydrocarbons.

55. The microbial oil according to any one of claims 44 to 54, comprising a PUFA having at least 0.5 area%.

56. The microbial oil according to claim 55, wherein the PUFA comprises a non-productive PUFA externally added.

57. The microbial oil according to any one of claims 44 to 56, wherein the microbial oil is crude oil.

58. The microbial oil according to any one of claims 44 to 57, wherein the microbial oil is yeast oil.

59. The microbial oil according to claim 58, wherein the yeast oil is an oil derived from yeast expressing a PUFA chain elongase gene.

60. The microbial oil according to claim 59, wherein the PUFA chain elongase gene is expressed by an inducible promoter.

61. The microbial oil according to claim 59 or 60, wherein
the PUFA chain elongase gene comprises
a) a polynucleotide encoding non-mammalian ELOVL4; or
b) a polynucleotide having a nucleotide sequence having at least 90% sequence identity to a nucleotide sequence of the polynucleotide encoding non-mammalian ELOVL4 and encoding a protein having PUFA chain elongation activity.

62. A method for producing a polyunsaturated fatty acid and/or a derivative thereof, the method comprising:
(A) transducing an expression system of a PUFA chain elongase into a cell;
(B) conditioning the cell transduced to a state of high VLC-PUFA content; and
(C) inducing expression of the PUFA chain elongase in the cell conditioned.

63. The method according to claim 62, wherein the cell is yeast.

64. The method according to claim 62 or 63, wherein the PUFA chain elongase is non-mammalian ELOVL4.

65. The method according to any one of claims 62 to 64, wherein a gene of the PUFA chain elongase is expressed by an inducible promoter.

66. The method according to claim 65, wherein the inducible promoter is galactose-inducible and an addition of galactose induces an expression of an elovl4 gene.

67. The method according to any one of claims 62 to 66, wherein step (B) is culturing in a culture medium comprising a polyunsaturated fatty acid having a carbon chain length of 18 to 22 and/or a derivative thereof.

68. The method according to any one of claims 62 to 67, wherein the expression of the PUFA chain elongase is induced in the culture solution having a turbidity value in a range of 1.5 to 11.

69. The method according to any one of claims 62 to 68, wherein a polyunsaturated fatty acid to be produced comprising a polyunsaturated fatty acid having at least 30 carbons and/or a derivative thereof.

70. The method according to claim 69, wherein
the polyunsaturated fatty acid having at least 30 carbons comprises at least one polyunsaturated fatty acid selected from the group consisting of the following:
(1) a polyunsaturated fatty acid having 30 carbons;
(2) a polyunsaturated fatty acid having 32 carbons;
(3) a polyunsaturated fatty acid having 34 carbons; and
(4) a polyunsaturated fatty acid having 36 carbons.

71. The method according to claim 70, wherein a content of one polyunsaturated fatty acid selected from the group consisting of (1) to (4) and/or a derivative thereof is at least 0.5 area%.

72. The method according to claim 70 or 71, wherein the polyunsaturated fatty acid having at least 30 carbons and/or the derivative thereof comprises the polyunsaturated fatty acids of (1) to (4) and/or the derivatives thereof at a content of at least 1.0 area%, at least 1.5 area%, at least 2.0 area%, or at least 2.5 area%.

73. The method according to any one of claims 70 to 72, wherein a ratio of a content of stearic acid and/or a derivative thereof to a total content of the polyunsaturated fatty acids of (1) to (4) and/or the derivatives thereof is less than 6.0, less than 5.0, less than 2.5, or less than 2.0.

74. The method according to any one of claims 70 to 73, wherein
the polyunsaturated fatty acid and/or the derivative thereof satisfies at least one of the following conditions:
(1) a ratio of a content of stearic acid and/or a derivative thereof to a content of the polyunsaturated fatty acid having 30 carbons and/or the derivative thereof is less than 50, less than 30, less than 20, or less than 10;
(2) a ratio of a content of stearic acid and/or a derivative thereof to a content of the polyunsaturated fatty acid having 32 carbons and/or the derivative thereof is less than 15, less than 10, or less than 5;
(3) a ratio of a content of stearic acid and/or a derivative thereof to a content of the polyunsaturated fatty acid having 34 carbons and/or the derivative thereof is less than 10, less than 5, or less than 2; and
(4) a ratio of a content of stearic acid and/or a derivative thereof to a content of the polyunsaturated fatty acid having 36 carbons and/or the derivative thereof is less than 20.

75. The method according to any one of claims 70 to 74, wherein the polyunsaturated fatty acid and/or the derivative thereof comprises an impurity having a chemical formula of C23H36O3.

76. Microbial oil produced by the method according to any one of claims 62 to 75.
